(19) **Europäisches Patentamt / European Patent Office / Office européen des brevets**

(11) **EP 2 970 414 B1**

(12) **EUROPEAN PATENT SPECIFICATION**

(45) Date of publication and mention of the grant of the patent:
**31.10.2018 Bulletin 2018/44**

(21) Application number: **14719989.7**

(22) Date of filing: **14.03.2014**

(51) Int Cl.:
**C07K 14/47** (2006.01)

(86) International application number:
**PCT/US2014/028650**

(87) International publication number:
**WO 2014/144303 (18.09.2014 Gazette 2014/38)**

(54) **FUSION PROTEINS AND METHODS FOR IDENTIFYING BROMODOMAIN INHIBITING COMPOUNDS**

FUSIONSPROTEINE UND VERFAHREN ZUR IDENTIFIZIERUNG VON BROMDOMÄNENHEMMENDEN VERBINDUNGEN

PROTÉINES HYBRIDES ET MÉTHODES D'IDENTIFICATION DE COMPOSÉS INHIBANT LE BROMODOMAINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.03.2013 US 201361798644 P**
**11.11.2013 US 201361902690 P**

(43) Date of publication of application:
**20.01.2016 Bulletin 2016/03**

(73) Proprietor: **Constellation Pharmaceuticals, Inc.**
**Cambridge, MA 02142 (US)**

(72) Inventors:
• **HUANG, Hon-Ren**
**Cambridge, Massachusetts 02142 (US)**
• **SIMS, Robert, J. III**
**Cambridge, Massachusetts 02142 (US)**
• **BELLON, Steven, Frank**
**Cambridge, Massachusetts 02142 (US)**

(74) Representative: **Lenthall, Joseph et al**
**Mewburn Ellis LLP**
**City Tower**
**40 Basinghall Street**
**London EC2V 5DE (GB)**

(56) References cited:
• **PANAGIS FILIPPAKOPOULOS ET AL: "Selective inhibition of BET bromodomains", NATURE, vol. 468, no. 7327, 24 September 2010 (2010-09-24), pages 1067-1073, XP055104608, ISSN: 0028-0836, DOI: 10.1038/nature09504**
• **CA FRENCH ET AL: "BRD NUT oncoproteins: a family of closely related nuclear proteins that block epithelial differentiation and maintain the growth of carcinoma cells", ONCOGENE, NATURE PUBLISHING GROUP, GB, vol. 27, 1 January 2008 (2008-01-01), pages 2237-2242, XP007919793, ISSN: 0950-9232, DOI: 10.1038/SJ.ONC.1210852 [retrieved on 2007-10-15]**
• **SEUL-KI LEE ET AL: "Genome-wide screen of human bromodomain-containing proteins identifies Cecr2 as a novel DNA damage response protein", MOLECULES AND CELLS, vol. 34, no. 1, 12 June 2012 (2012-06-12), pages 85-91, XP055130772, ISSN: 1016-8478, DOI: 10.1007/s10059-012-0112-4**
• **S. PICAUD ET AL: "PFI-1, a Highly Selective Protein Interaction Inhibitor, Targeting BET Bromodomains", CANCER RESEARCH, vol. 73, no. 11, 1 June 2013 (2013-06-01), pages 3336-3346, XP055130754, ISSN: 0008-5472, DOI: 10.1158/0008-5472.CAN-12-3292**

## Description

### BACKGROUND

Chromatin is a complex combination of DNA and proteins. It is found inside the nuclei of eukaryotic cells and is divided between heterochromatin (condensed) and euchromatin (extended) forms. Histones are the chief protein components of chromatin, acting as spools around which DNA winds. The functions of chromatin are to package DNA into a smaller volume to fit in the cell, to strengthen the DNA to allow mitosis and meiosis, and to serve as a mechanism to control expression and DNA replication. The chromatin structure is controlled by a series of post-translational modifications to histone proteins, notably histones H3 and H4, and most commonly within the "histone tails" that extend beyond the core nucleosome structure. Histone tails tend to be free for protein-protein interaction and are also the portion of the histone most prone to post-translational modification. These modifications include acetylation, methylation, phosphorylation, ubiquitinylation and SUMOylation. These epigenetic marks are written and erased by specific enzymes that place the tags on specific residues within the histone tail, thereby forming an epigenetic code, which is then interpreted by the cell to allow gene specific regulation of chromatin structure and thereby transcription.

Of all classes of proteins, histones are amongst the most susceptible to post-translational modification. Histone modifications are dynamic, as they can be added or removed in response to specific stimuli, and these modifications direct both structural changes to chromatin and alterations in gene transcription. Distinct classes of enzymes, namely histone acetyltransferases (HATs) and histone deacetylases (HDACs), acetylate or de-acetylate specific histone lysine residues (Struhl K., Genes Dev., 1989, 12, 5, 599-606).

Covalent modification of histones is a fundamental mechanism of control of gene expression, and one of the major epigenetic mechanisms at play in eukaryotic cells (Kouzarides, Cell, 128, 693-705 (2007)). Because distinct transcriptional states define fundamental cellular processes, such as cell type specification, lineage commitment, cell activation and cell death, their aberrant regulation is at the core of a range of diseases (Medzhitov et al., Nat. Rev. Immunol., 9, 692-703 (2009); Portela et al., Nat. Biotech., 28, 1057-1068 (2010)). A fundamental component of the epigenetic control of gene expression is the interpretation of histone modifications by proteins that harbor specialized motifs that bind to such modifications. Among them, bromodomains have evolved to bind to acetylated histones and by so doing they represent fundamental links between chromatin structure and gene transcription (Fillipakoppoulos et al., Cell, 149, 214-231 (2012)).

Bromodomains, which are approximately 110 amino acids long, are found in a large number of chromatin-associated proteins and have been identified in approximately 70 human proteins, often adjacent to other protein motifs (Jeanmougin F., et al., Trends Biochem. Sci., 1997, 22, 5, 151-153; and Tamkun J.W., et al., Cell, 1992, 7, 3, 561-572). Interactions between bromodomains and modified histones may be an important mechanism underlying chromatin structural changes and gene regulation. Bromodomain-containing proteins have been implicated in disease processes including cancer, inflammation and viral replication. *See, e.g.*, Prinjha et al., Trends Pharm. Sci., 33(3):146-153 (2012) and Muller et al., Expert Rev., 13(29):1-20 (September 2011).

Cell-type specificity and proper tissue functionality requires the tight control of distinct transcriptional programs that are intimately influenced by their environment. Alterations to this transcriptional homeostasis are directly associated with numerous disease states, most notably cancer, immuno-inflammation, neurological disorders, and metabolic diseases. Bromodomains reside within key chromatin modifying complexes that serve to control distinctive disease-associated transcriptional pathways. This is highlighted by the observation that mutations in bromodomain-containing proteins are linked to cancer, as well as immune and neurologic dysfunction. Hence, the selective inhibition of bromodomains across the family creates varied opportunities as novel therapeutic agents in human dysfunction.

There is a need for treatments for cancer, immunological disorders, and other bromodomain related diseases. As such, methods for identifying compounds that are bromodomain inhibiting compounds are needed.

### SUMMARY

Provided herein is a method for determining whether a test compound is a bromodomain inhibiting compound comprising (a) contacting a cell which contains a fusion protein, wherein a plurality of fusion proteins are capable of forming foci, with the test compound; and (b) determining whether the test compound causes an increase in formation of fusion protein foci, wherein an increase in formation of foci indicates that the test compound is a bromodomain inhibiting compound, wherein each fusion protein comprises at least one bromodomain and a reporter module which is a fluorescent protein that is capable of multimerizing, and wherein the reporter module is EGFP, TurboGFP dsRed2, dsRed-Express2 or ZsGreen.

In certain embodiments of any of the fusion proteins, the fusion protein comprises a nuclear localization signal (NLS). In certain embodiments, the NLS is the SV40 Large T-antigen NLS or the NLS of nucleoplasmin.

In certain embodiments of any of the fusion proteins, the chromatin binding module is located 5' of the reporter module. In certain embodiments of any of the fusion proteins, the chromatin binding module is located 3' of the reporter module.

The chromatin binding module is a bromodomain module.

In certain embodiments of any of the fusion proteins, the at least one bromodomain module comprises at least one bromodomain of any one of BRG1, PCAF/KAT2B, BAZ2B, BRD1, BRD8, BRFP1, BRFP3, BRG1, CBP/CREBBP, PCAF/KAT2B, TRIM24 and/or ZMYND8. In certain embodiments, the at least one bromodomain module comprises at least one bromodomain of any one of BRD2, BRD3, BRD4, BRD9, BRDT, and/or BRG1. In certain embodiments, the at least one bromodomain module comprises at least one bromodomain of any one of BRG1, BRPF1, CECR2, PCAF, and/or TAF1. In certain embodiments, the at least one bromodomain module comprises at least one bromodomain of BRD4 and/or BRD9.

In certain embodiments of any of the fusion proteins, the bromodomain polypeptide comprises the amino acid sequence of any one of BRG1, PCAF/KAT2B, BAZ2B, BRD1, BRD8, BRFP1, BRFP3, BRG1, CBP/CREBBP, PCAF/KAT2B, TRIM24, and/or ZMYND8, or a fragment thereof comprising at least one bromodomain module. In certain embodiments, the bromodomain polypeptide comprises the amino acid sequence of any one of BRD2, BRD3, BRD4, BRD9, BRDT, and/or BRG1, or a fragment thereof comprising at least one bromodomain module. In certain embodiments, the bromodomain polypeptide comprises the amino acid sequence of any one of BRG1, BRPF1, CECR2, PCAF, and/or TAF1, or a fragment thereof comprising at least one bromodomain module. In certain embodiments, the bromodomain polypeptide comprises the amino acid sequence of BRD4 and/or BRD9, or a fragment thereof comprising at least one bromodomain module. In certain embodiments, the bromodomain polypeptide comprises a full length bromodomain polypeptide.

The fusion protein is capable of multimerizing. In certain embodiments, the fusion protein is capable of forming a dimer, a trimer or a tetramer. In certain embodiments, the fusion protein is capable of forming a dimer. In certain embodiments, the fusion protein is capable of forming a tetramer.

Disclosed herein is a nucleic acid sequence (e.g., DNA or RNA) encoding a fusion protein described herein. Also disclosed is an expression cassette comprising the nucleic acid sequence. Also disclosed is a cell comprising the expression cassette.

Also disclosed is a cell comprising a fusion protein described herein.

In certain embodiments, the cell is a CHO-K1, COS-7, HEK293, HEK293T, HEK293FT, HeLa, MDCK or U2OS cell. In certain embodiments, the cell is a COS-7, HeLa or U2OS cell.

Also disclosed is a method for determining whether a test compound is a bromodomain inhibiting compound comprising (a) contacting a cell described herein that comprises a fusion protein comprising at least one chromatin binding module and at least one reporter module with the test compound and (b) determining whether the test compound induces relocalization of the fusion protein and/or increases formation of fusion protein foci, wherein relocalization of the fusion protein and/or an increase in formation of foci indicates that the test compound is a bromodomain inhibiting compound.

Also disclosed is a kit comprising the fusion protein, nucleic acid, expression cassette or cell described herein.

## BRIEF DESCRIPTION OF THE FIGURES

Figure 1 depicts certain fusion proteins of the invention.

Figure 2 depicts results demonstrating that fusion proteins form dots/foci in response to inhibitors when the reporter module comprises a protein that could form multimers (e.g., dimers or tetramers).

Figure 3 depicts the localization of ZsGreen-BRD4 fusion protein (A) and ZsGreen-BRD4 fusion protein with point mutations in both bromodomain modules which prevent binding to chromatin (B). Mutant ZsGreeen-BRD4 fusion protein was localized to dots/foci in the nucleus even in the absence of inhibitors (Figure 3B) while wild type fusion proteins showed diffuse localization under the same condition (Figure 3A).

Figure 4 depicts further results demonstrating that inhibiting compounds prevented bromodomain binding to the chromatin, which was stained with Hoechst 33342.

Figure 5 depicts results showing the fast kinetics of fusion protein relocalization. The effects of inhibitors could be monitored in real-time by following the foci formation.

Figure 6 depicts results demonstrating that relocalization and foci formation was a titratable phenotype that could be used to determine the potency of inhibitors in cellular settings.

Figure 7 presents one, non-limiting model regarding a possible mechanism of foci formation in response to inhibitors.

Figure 8A-B depicts the localization of ZsGreen-BRD2 fusion protein in the absence (A) and presence (B) of the BRD2, BRD3, BRD4, and BRDT bromodomain inhibiting compound JQ1. JQ1 treatment resulted in disruption of the interaction of ZsGreen-BRD2 fusion protein with chromatin and the formation of dots/foci.

Figure 9A-B depicts the localization of ZsGreen-BRD3 fusion protein in the absence (A) and presence (B) of JQ1. JQ1 treatment resulted in disruption of the interaction of ZsGreen-BRD3 fusion protein with chromatin and the formation of dots/foci.

Figure 10A-B depicts the localization of BRD9-ZsGreen fusion protein in the absence (A) and presence (B) of the BRD9 bromodomain inhibiting compounds BDi-B and BDi-C. Inhibitor treatment resulted in disruption of the interaction of BRD9-ZsGreen fusion protein with chromatin and the formation of dots/foci.

Figure 11A-B depicts the localization of BRD9-ZsGreen fusion protein (A) and BRD9-ZsGreen fusion protein with point mutation (N216Y) in the bromodomain modules which prevent binding to chromatin (B). Mutant BRD9-ZsGreen lacking the binding affinity toward chromatin formed fluorescent dots even in the absence of chromatin inhibiting compounds.

Figure 12A-D depicts the localization of BRD9-mVenus fusion protein with DMSO (A) and BRD9 bromodomain inhibiting compound BDi-D (B), and the localization of mutant BRD9-mVenus with point mutation (N216Y) in the bromodomain modules which prevent binding to chromatin (C) treated with DMSO (D). Neither bromodomain inhibiting compound or the bromodomain mutation resulted in the formation of fluorescent dots/foci.

Figure 13A-C depicts the localization of NLS-CECR2.BD-ZsGreen fusion protein (A) in the absence and presence (B) of the CECR2 bromodomain inhibiting compound BDi-E. BDi-E treatment results in formation of NLS-CECR2.BD-ZsGreen foci when not bound to the chromatin.

Figure 14 depicts results demonstrating other detection methods, which included biochemical methods such as employing fractionation and Western blotting.

Figure 15A-B depicts the localization ofNLS-TAF1.BD1.BD2-ZsGreen (A) in the absence and presence (B) of a TAF1 bromodomain inhibiting compound, BDi-F. The results showed that inhibitors disrupted the binding of fusion proteins to chromatin, and fusion proteins formed foci after being released from the chromatin.

Figure 16 depicts the localization of mutant NLS-TAF1.BD1.BD2-ZsGreen protein with point mutations in both bromodomain domains which prevent binding to chromatin (A). The results showed that mutant fusion proteins formed foci even in the absence of inhibiting compounds (B).

Figure 17A-B depicts the localization of BAZ2B-BRD9-ZsGreen fusion protein (BRD9 bromodomain polypeptide in which the BRD9 bromodomain module had been replaced with the bromodomain module of BAZ2B) (A) in the absence and presence (B) of the BAZ2B bromodomain inhibiting compound BDi-G. BDi-G treatment resulted in disruption of the interaction of BAZ2B-BRD9-ZsGreen fusion protein with chromatin and the formation of dots/foci.

Figure 18 depicts the localization of PCAF-BRD9-ZsGreen fusion protein (BRD9 bromodomain polypeptide in which the BRD9 bromodomain module had been replaced with the bromodomain module of PCAF) (A) in the absence and presence (B) of the PCAF bromodomain inhibiting compound BDi-H. BDi-H treatment resulted in disruption of the interaction of PCAF-BRD9-ZsGreen fusion protein with chromatin and the formation of dots/foci.

## DETAILED DESCRIPTION

Disclosed herein are fusion proteins that comprise at least one chromatin binding module and at least one reporter module, wherein a plurality of fusion proteins are capable of forming foci. These fusion proteins can be used to determine whether a test compound is a chromatin-inhibiting compound. Also provided is an assay that utilizes the fusion proteins to determine whether a test compound is a chromatin-inhibiting compound. It is believed that in an untreated state, the fusion protein binds chromatin through interaction between the chromatin binding module and the chromatin. For example, bromodomain modules bind to the acetylated chromatin. When the chromatin binding module binding site is blocked by an inhibitor, the fusion protein dissociates from chromatin and forms foci. A cellular target engagement assay can be used to monitor formation of foci in a dose dependent manner. The foci in the nucleus of a cell can be visualized and quantified by high content microscopy using, *e.g.*, fluorescent detection, to determine the efficacy of a test compound as a chromatin-inhibiting compound.

### General Techniques

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., ed., 1994); "A Practical Guide to Molecular Cloning" (Perbal Bernard V., 1988).

Oligonucleotides, polynucleotides, peptides, polypeptides and small molecules employed or described in the present invention can be generated using standard techniques known in the art.

*Definitions*

The term "chromatin binding module" as used herein refers to the sequence of a region and/or domain of a chromatin binding polypeptide that interacts with chromatin. Chromatin binding modules can include, but are not limited to, at least one of the following domains: ADD, ankyrin repeats, bromodomain, chromodomain, MBT, PHD finger, PWWP, Tudor, WD40 or Zf-CW (also, *see* Miyong Yun et al. Cell Research (2011) 21:564-578.

The term "chromatin binding polypeptide" as used herein refers to a native sequence chromatin binding polypeptide, polypeptide variants of a native sequence polypeptide and polypeptide variants (which are further defined herein). The chromatin binding polypeptide described herein may be that which is isolated from a variety of sources, such as from human tissue types or from another source, or prepared by recombinant or synthetic methods. A "native sequence chromatin binding polypeptide" comprises a polypeptide having the same amino acid sequence as the corresponding chromatin binding polypeptide derived from nature. A chromatin binding polypeptide comprises at least one chromatin binding module.

"Chromatin binding polypeptide variant", or variations thereof, means a chromatin binding polypeptide, generally an active chromatin binding polypeptide, as defined herein having at least about 80% amino acid sequence identity with any of the native sequence chromatin binding polypeptide sequences as disclosed herein. Such chromatin binding polypeptide variants include, for instance, chromatin binding polypeptides wherein one or more amino acid residues are added, or deleted, at the N- or C-terminus of a native amino acid sequence. Ordinarily, a chromatin binding polypeptide variant will have at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to a native sequence chromatin binding polypeptide sequence. Ordinarily, chromatin binding variant polypeptides are at least about 10 amino acids in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 amino acids in length, or more. Optionally, chromatin binding variant polypeptides will have no more than one conservative amino acid substitution as compared to a native chromatin binding polypeptide sequence, alternatively no more than 2, 3, 4, 5, 6, 7, 8, 9, or 10 conservative amino acid substitution as compared to the native chromatin binding polypeptide sequence.

The term "bromodomain module" refers to the sequence of a bromodomain of a bromodomain polypeptide that interacts with chromatin. In certain embodiments, the bromodomain module comprises a full length bromodomain. For a general review on bromodomain structures and function, *see*, *e.g.*, Filippakopoulos et al., Cell 149, 214-231 (2012).

The term "bromodomain polypeptide" as used herein refers to a native sequence chromatin binding polypeptide, polypeptide variants of a native sequence polypeptide and polypeptide variants (which are further defined herein). The bromodomain polypeptide described herein may be that which is isolated from a variety of sources, such as from human tissue types or from another source, or prepared by recombinant or synthetic methods. A "native sequence bromodomain polypeptide" comprises a polypeptide having the same amino acid sequence as the corresponding bromodomain polypeptide derived from nature. A bromodomain polypeptide comprises at least one bromodomain module.

"Bromodomain polypeptide variant", or variations thereof, means a bromodomain polypeptide, generally an active bromodomain polypeptide, as defined herein having at least about 80% amino acid sequence identity with any of the native sequence bromodomain polypeptide sequences as disclosed herein. Such bromodomain polypeptide variants include, for instance, bromodomain polypeptides wherein one or more amino acid residues are added, or deleted, at the N- or C-terminus of a native amino acid sequence. Ordinarily, a bromodomain polypeptide variant will have at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to a native sequence chromatin binding polypeptide sequence. Ordinarily, bromodomain variant polypeptides are at least about 10 amino acids in length, alternatively at least about 20, 30, 40, 50, 60, 70, 80, 90, 100, 110, 120, 130, 140, 150, 160, 170, 180,190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 amino acids in length, or more. Optionally, bromodomain variant polypeptides will have no more than one conservative amino acid substitution as compared to a native bromodomain polypeptide sequence, alternatively no more than 2, 3, 4, 5, 6, 7, 8, 9, or 10 conservative amino acid substitution as compared to the native bromodomain polypeptide sequence.

The term "reporter module" refers to an amino acid sequence that allows for detection of the change of localization or molecular properties of the fusion protein in response to chemical compounds which prevent chromatin binding of the chromatin binding module. For example, the reporter module introduces a measurable property difference between 'bound' and 'unbound' states. For example, an indirect fluorescence method can be used to detect TAG-BM-RM using antibodies against the TAG [BM: binding module, RM: reporter module].

The term "nucleic acid" refers to deoxyribonucleotides or ribonucleotides and polymers thereof in either single- or double-stranded form, made of monomers (nucleotides) containing a sugar, phosphate and a base that is either a purine or pyrimidine. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues.

The term "nucleotide sequence" refers to a polymer of DNA or RNA that can be single-stranded or double-stranded, optionally containing synthetic, non-natural or altered nucleotide bases capable of incorporation into DNA or RNA polymers. The terms "nucleic acid," "nucleic acid molecule," or "polynucleotide" are used interchangeably.

An "isolated" polypeptide is one which had been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of polypeptide purity, *see*, *e.g.*, Flatman et al., J. Chromatogr. B 848:79-87 (2007).

An "isolated" nucleic acid refers to a nucleic acid molecule that had been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code had been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "substantial identity" in the context of a peptide indicates that a peptide comprises a sequence with at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, or 94%, or even 95%, 96%, 97%, 98% or 99%, sequence identity to the reference sequence over a specified comparison window. In certain embodiments, optimal alignment is conducted using the homology alignment algorithm of Needleman and Wunsch (Needleman and Wunsch, JMB, 48, 443 (1970)). An indication that two peptide sequences are substantially identical is that one peptide is immunologically reactive with antibodies raised against the second peptide. Thus, a peptide is substantially identical to a second peptide, for example, where the two peptides differ only by a conservative substitution. Thus, certain embodiments provide nucleic acid molecules that are substantially identical to the nucleic acid molecules described herein.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

"Operably-linked" refers to the association of nucleic acid sequences on single nucleic acid fragment so that the function of one of the sequences is affected by another. For example, a regulatory DNA sequence is said to be "operably linked to" or "associated with" a DNA sequence that codes for an RNA or a polypeptide if the two sequences are situated such that the regulatory DNA sequence affects expression of the coding DNA sequence (e.g., that the coding sequence or functional RNA is under the transcriptional control of the promoter). Coding sequences can be operably-linked to regulatory sequences in sense or antisense orientation. Nucleic acid is "operably linked" when it is placed into a functional relationship with another nucleic acid sequence. Generally, "operably linked" means that the DNA sequences being linked are contiguous. However, enhancers do not have to be contiguous. Linking is accomplished by ligation at convenient restriction sites. If such sites do not exist, the synthetic oligonucleotide adaptors or linkers are used in accordance with conventional practice. Additionally, multiple copies of the nucleic acid encoding enzymes may be linked together in the expression vector. Such multiple nucleic acids may be separated by linkers.

"Expression" refers to the transcription and/or translation of an endogenous gene or a transgene in cells. For example, in the case of antisense constructs, expression may refer to the transcription of the antisense DNA only. In addition, expression refers to the transcription and stable accumulation of sense (mRNA) or functional RNA. Expression may also refer to the production of protein.

"Expression cassette" as used herein means a DNA sequence capable of directing expression of a particular nucleotide sequence in an appropriate host cell, comprising a promoter operably linked to the nucleotide sequence of interest that is operably linked to termination signals. It also typically comprises sequences required for proper translation of the nucleotide sequence. The expression cassette comprising the nucleotide sequence of interest may be chimeric, meaning that at least one of its components is heterologous with respect to at least one of its other components. The expression cassette may also be one that is naturally occurring but has been obtained in a recombinant form useful for heterologous expression. Such expression cassettes will comprise the transcriptional initiation region linked to a nucleotide sequence of interest. Such an expression cassette may be provided with a plurality of restriction sites for insertion of the gene of interest to be under the transcriptional regulation of the regulatory regions. The expression cassette may additionally contain selectable marker genes.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The terms "measurable affinity" and "measurably inhibit," as used herein, refer to a measurable reduction in activity of a bromodomain between: (i) a sample comprising a bromodomain inhibitor or composition thereof and such bromodomain, and (ii) an equivalent sample comprising such bromodomain, in the absence of said compound, or composition thereof.

The phrase "substantially similar," as used herein, refers to a sufficiently high degree of similarity between two numeric values (generally one associated with a molecule and the other associated with a reference/comparator molecule) such that one of skill in the art would consider the difference between the two values to not be of statistical significance within the context of the biological characteristic measured by said values (e.g., Kd values). The difference between said two values may be, for example, less than about 20%, less than about 10%, and/or less than about 5% as a function of the reference/comparator value. The phrase "substantially normal" refers to substantially similar to a reference (e.g., normal reference).

The phrase "substantially different," refers to a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (e.g., Kd values). The difference between said two values may be, for example, greater than about 10%, greater than about 20%, greater than about 30%, greater than about 40%, and/or greater than about 50% as a function of the value for the reference/comparator molecule.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of polynucleotide analysis or protocol, one may use the results of the polynucleotide expression analysis or protocol to determine whether a specific therapeutic regimen should be performed.

An "individual" or "subject" is a mammal. Mammals include, but are not limited to, domesticated animals (*e.g.*, cows, sheep, cats, dogs, and horses), primates (*e.g.*, humans and non-human primates such as monkeys), rabbits, and rodents (e.g., mice and rats). In certain embodiments, the individual or subject is a human.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies are used to delay development of a disease or to slow the progression of a disease.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

Recitation of ranges of values herein are merely intended to serve as a shorthand method of referring individually to each separate value falling within the range, unless otherwise indicated herein, and each separate value is incorporated into the specification as if it were individually recited herein.

As is understood by one skilled in the art, reference to "about" a value or parameter herein includes (and describes) embodiments that are directed to that value or parameter per se. For example, description referring to "about X" includes description of "X".

The use of the terms "a" and "an" and "the" and similar terms in the context of describing embodiments of invention are to be construed to cover both the singular and the plural, unless otherwise indicated herein or clearly contradicted by context. The terms "comprising," "having," "including," and "containing" are to be construed as open-ended terms (*i.e.*, meaning "including, but limited to") unless otherwise noted. It is understood that aspect and embodiments of the invention described herein include "consisting" and/or "consisting essentially of' aspects and embodiments.

## Methods of Screening and Fusion Proteins

Provided herein are methods of screening compounds to identify those that modulate a polypeptide comprising a bromodomain module and compositions useful in the methods. In particular, provided herein are fusion proteins comprising at least one chromatin binding module and at least one reporter module, and methods of screening compounds using a fusion protein that comprise at least one chromatin binding module and at least one reporter module.

Provided herein are fusion proteins comprising at least one chromatin binding module and at least one reporter module, wherein a plurality of fusion proteins are capable of forming foci.

In one aspect, provided herein method for determining whether a test compound is a bromodomain inhibiting compound comprising (a) contacting a cell described herein that comprises a fusion protein comprising at least one chromatin binding module and at least one reporter module with the test compound and (b) determining whether the test compound changes the distribution pattern of the fusion protein and/or increases formation of fusion protein foci, wherein a change in distribution pattern and/or an increase in formation of foci indicates that the test compound is a bromodomain inhibiting compound. In some embodiments, the chromatin binding module is a bromodomain module.

In some aspect, provided herein are methods of identifying a compound capable of specifically binding a chromatin binding module and inhibiting its interaction with chromatin, said method comprising (a) contacting a cell comprising a fusion protein, wherein the fusion protein comprises at least one chromatin binding module and at least one reporter module, with a test compound, (b) determining the distribution of reporter signal in the cell comprising the fusion protein in the presence and absence of the test compound, wherein an increase in reporter signal foci in the presence of the test compound compared to in the absence of the test compound indicates that the test compound is a compound capable of specifically binding a chromatin binding module and inhibiting its interaction with chromatin. In some embodiments, the chromatin binding module is a bromodomain module.

In some embodiments, the fusion protein comprises about any of 1, 2, 3, 4, 5, or 6 chromatin binding modules. In some embodiments, the fusion protein comprises about one chromatin binding module. In some embodiments, the fusion protein comprises two chromatin binding modules. In some embodiments, the fusion protein comprises at least one chromatin binding module from a first chromatin binding polypeptide and at least one chromatin binding module from a second chromatin binding polypeptide. In some embodiments, the fusion protein comprises one chromatin binding module from a first chromatin binding polypeptide and one chromatin binding module from a second chromatin binding polypeptide.

In some embodiments of any of the methods and fusion proteins, the at least one chromatin binding module (e.g., bromodomain module) is in its endogenous and/or native context. In some embodiments, in the endogenous context, the fusion protein comprises the amino acid sequence of a native chromatin binding polypeptide or a fragment of the native chromatin binding polypeptide comprising at least one chromatin binding module and at least one reporter module. For example, in the endogenous context, the fusion protein comprises the amino acid sequence of the native bromodomain polypeptide, *e.g.*, BRG1, which comprises at least one bromodomain, and at least one reporter construct. In some embodiments, the fusion protein comprises the full-length sequence of the chromatin binding polypeptide. In some embodiments, the fusion protein comprises a fragment of the chromatin binding polypeptide comprising the chromatin binding module. In some embodiments, the fusion protein comprises at least about 50, 60, 70, 80, and 90% of the chromatin binding polypeptide and includes the chromatin binding module. In some embodiments, the chromatin binding module is a bromodomain module. In some embodiments, the chromatin binding polypeptide is a bromodomain polypeptide.

In some embodiments of any of the methods and fusion proteins, at least one chromatin binding module (e.g., bromodomain module) is in an exogenous and/or nonnative context. The chromatin binding module (e.g., bromodomain module) in an exogenous and/or nonnative context includes, but is not limited to (i) a chromatin binding module in a different amino acid position/context in a chromatin binding polypeptide compared to a native chromatin binding polypeptide, (ii) a chromatin binding module of a first chromatin binding polypeptide in the context of a second chromatin binding polypeptide, and/or (iii) a chromatin binding module in the context of an unrelated polypeptide (e.g., non-chromatin binding polypeptide). In some embodiments, the fusion protein comprises at least one chromatin binding module in a different amino acid position/context in a chromatin binding polypeptide compared to a native chromatin binding polypeptide and at least one chromatin binding module in a same or similar amino acid position/context in a chromatin binding polypeptide compared to a native chromatin binding polypeptide. In some embodiments, the chromatin binding module is a bromodomain module. In some embodiments, the chromatin binding polypeptide is a bromodomain polypeptide.

In certain embodiments of any of the methods and fusion proteins, the fusion protein comprises a first chromatin binding polypeptide, wherein one or more of the chromatin binding modules of the first chromatin binding polypeptide have been substituted and/or replaced with at least one chromatin binding module of a second chromatin binding polypeptide. For example, Example 7 presents certain embodiments of the invention in the fusion protein comprises a fluorescent protein and human BRD9 coding DNA sequence, in which the bromodomain coding sequence was replaced with the bromodomain sequence of human BAZ2B or human PCAF/KAT2B.

In some embodiments of any of the methods and fusion proteins, the fusion protein comprises a chromatin binding polypeptide with multiple chromatin binding modules, wherein chromatin binding modules have sufficient affinity for chromatin. In certain instances, native proteins may contain multiple chromatin binding modules, and inhibition of one module does not release the fusion protein from chromatin. On the other hand, certain chromatin binding modules may not bind to the chromatin sufficiently when expressed as a small module by itself. In these cases, a chimeric protein with a backbone from another chromatin binding polypeptide can be used to solve these problems. To assess whether a chromatin binding polypeptide is suitable for the relocalization/foci formation assay, a mutation can be introduced to the binding pocket of the chromatin binding module to disrupt the binding ability. If the mutant fusion protein can still bind to the chromatin and does not show a different distribution pattern compared to the wild type fusion protein, it is likely the chromatin binding polypeptide cannot be used in the method described in this patent. This type of result indicates that regions other than the chromatin binding module of interest may also contribute to the affinity for chromatin. To resolve this issue, a chimeric chromatin binding polypeptide having a backbone that does not have affinity for chromatin can be used to establish an assay.

In some embodiments of any of the methods and fusion proteins, the fusion protein comprises a first chromatin binding polypeptide, wherein one or more of the chromatin binding modules has been duplicated and/or repeated. In other embodiments, the fusion protein comprises a first chromatin binding polypeptide, wherein at least one chromatin binding module from a second chromatin binding polypeptide. In some embodiments of any of the methods and fusion proteins, the chromatin binding module is a bromodomain module. In some embodiments, the chromatin binding polypeptide is a bromodomain polypeptide.

In some embodiments of any of the methods and fusion proteins, the chromatin binding module is a bromodomain module, PHD finger module, chromodomain module, MBT domain module, tudor domain module, PWWP domain module, ADD domain module, Zf-CW domain module, ankyrin repeat module and/or WD40 module.

In some embodiments of any of the methods and fusion proteins, the chromatin binding module is a bromodomain module. In some embodiments, the bromodomain module is an amino acid sequence characterized by a conserved fold that comprises a left-handed bundle of four α helices (αZ, αA, αB, αC), linked by loop regions of variable length (ZA and BC loops). In some embodiments, the bromodomain module comprises a histone ε-N-acetylation of lysine residues (Kac) binding site. In some embodiments, the bromodomain recognizes Kac by a central deep hydrophobic cavity, where it is anchored by a hydrogen bond to an asparagine residue. In some embodiments, the at least one bromodomain module comprises at least one bromodomain of any one of ASH1L, ATAD2, BAZ1A, BAZ1B, BAZ2A, BAZ2B, BRD1, BRD2, BRD3, BRD4, BRD7, BRD8, BRD9, BRDT, BRPF1, BRPF3, BRWD3, CECR2, CREBBP, EP300, FALZ, GCN5L2, KIAA1240, LOC93349, MLL, PB, PCAF, PHIP, PRKCBP1, SMARCA2, SMARCA4, SP100, SP110, SP140, TAF1, TAF1L, TIF1a, TRIM28, TRIM33, TRIM66, WDR9, ZMYND11, and/or MLL4. In some embodiments, the bromodomain module comprises at least one bromodomain of a protein in Table 1. In some embodiments, the bromodomain module comprises the sequence identified in Table 1 as a Bromodomain of UniProt SEQ ID (the UniPort sequences, including canonical sequences, are the sequences as accessed on November 1, 2013. In certain embodiments, the at least one bromodomain module comprises at least one bromodomain of any of BRG1, PCAF/KAT2B, BAZ2B, BRD1, BRD8, BRFP1, BRFP3, BRG1, CBP/CREBBP, PCAF/KAT2B, TRIM24, and/or ZMYND8. In certain embodiments, the at least one bromodomain module comprises at least one bromodomain of any one of BRD2, BRD3, BRD4, BRD9, BRDT and BRG1. In certain embodiments, the at least one bromodomain module comprises at least one bromodomain of any one of BRG1, BRPF1, CECR2, PCAF, and/or TAF1. In certain embodiments, the at least one bromodomain module comprises a bromodomain of BRD4 and/or BRD9. In some embodiments, the fusion protein comprises about any of 1, 2, 3, 4, 5, or 6 bromodomain modules.

Table 1 Bromodomain Polypeptides and Bromodomain Modules

| Protein | Name | Alias | Bromodomain of UniProt SEQ | UniProt SEQ ID |
|---------|------|-------|---------------------------|----------------|
| ASH1L | ash1 (absent, small, or homeotic)-like | ASH1, KMT2H | aa 2463-2533 | Q9NR48 |
| ATAD2 | Two AAA domain containing protein | ANCCA | aa 1001-1071 | Q6PL18 |
| BAZ1A | Bromodomain adjacent to zinc finger domain, 1A | ACF1, WALp1, WCRF180 | aa 1446-1516 | Q9NRL2 |
| BAZ1B | Bromodomain adjacent to zinc finger domain, 1B | WSTF, WBSCR9 | aa 1356-1426 | Q9UIG0 |
| BAZ2A | Bromodomain adjacent to zinc finger domain, 2A | TIP5, WALp3 | aa 1810-1880 | Q9UIF9 |
| BAZ2B | Bromodomain adjacent to zinc finger domain, 2B | WALp4 | aa 2077-2147 | Q9UIF8 |
| BRD1 | Bromodomain-containing protein 1 | BRL, BRPF2 | aa 579-649 | 095696 |
| BRD2 | Bromodomain-containing protein 2 | FSH, RING3 | aa 91-163 aa 364-436 | P25440 |
| BRD3 | Bromodomain-containing protein 3 | ORFX, RING3L | aa 51-123 aa 326-398 | Q15059 |
| BRD4 | Bromodomain-containing protein 4 | CAP, MCAP, HUNK1 | aa 75-147 aa 368-440 | O60885 |
| BRD7 | Bromodomain-containing protein 7 | BP75, NAG4, CELTIX1 | aa 148-218 | Q9NPI1 |
| BRD8 | Bromodomain-containing protein 8 | SMAP, SMAP2 | aa 724-794 aa 1120-1190 | Q9H0E9-2 |
| BRD9 | Bromodomain-containing protein 9 | | aa 153-223 | Q9H8M2 |
| BRDT | Bromodomain-containing protein, testis specific | BRD6 | aa 44-116 aa 287-359 | Q58F21 |
| BRPF1 | Bromodomain- and PHD finger-containing protein 1A | BR140, Peregrin | aa 645-715 | P55201-1 |
| BRPF3 | Bromodomain- and PHD finger-containing protein 3 | | aa 606-676 | Q9ULD4 |
| BRWD3 | Bromodomain-containing disrupted in leukemia | BRODL | aa 1158-1228 aa 1317-1412 | Q6RI45 |
| CECR2 | Cat eye syndrome chromosome region | | aa 451-521 | Q9BXF3 |

(continued)

| Protein | Name | Alias | Bromodomain of UniProt SEQ | UniProt SEQ ID |
|---|---|---|---|---|
| CREBBP | CREB-binding protein | CBP, KAT3A | aa 1103-1175 | Q92793 |
| EP300 | E1A-binding p300 | p300, KAT3B | aa 1067-1139 | Q09472 |
| FALZ | Fetal Alzheimer antigen | BPTF, FAC1 | aa 2944-3014 | Q12830 |
| GCN5L2 | General control of amino acid synthesis 5-like 2 | KAT2A, GCN5 | aa 745-815 | Q92830 |
| KIAA1240 | KIAA1240 | ATAD2B | aa 975-1045 | Q9ULI0 |
| LOC93349 | SP140-like | SP140L | aa 796-829 | Q13342 |
| MLL | Myeloid/lymphoid or mixed lineage leukemia (trithorax homolog, Drosophila) | HRX, TRX1, CXXC7, ALL-1 | aa 1703-1748 | Q03164 |
| PB1 | Polybromo 1 | PBRM1, BAF180 | aa 63-134 aa 200-270 aa 400-470 aa 538-608 aa 676-746 aa 792-862 | Q86U86 |
| PCAF | P300/CBP-associated factor | KAT2B | aa 740-810 | Q92831 |
| PHIP | Pleckstrin homology domain-interacting protein | WDR11, ndrp | aa 1176-1246 aa 1333-1403 | Q8WWQ0 |
| PRKCBP1 | Protein kinase C-binding 1 | ZMYND8, RACK7 | aa 165-235 | Q9ULU4 |
| SMARCA2 | SWI/SNF-related matrix-associated actin-dependent regulator of chromatin a2 | BRM, SNF2L2 | aa 1419-1489 | P51531 |
| SMARCA4 | SWI/SNF-related matrix-associated actin-dependent regulator of chromatin a4 | BRG1, SNF2L4, SNF2LB | aa 1477-1547 | P51532 |
| SP100 | Nuclear antigen Sp100 | | aa 761-876 | P23497-4 |
| SP110 | Nuclear antigen Sp110 A, nuclear antigen Sp110C | IPR1 | aa 581-676 | Q9HB58 |
| SP140 | SP140 nuclear body | LYSP100 | aa 796-829 | Q13342 |
| TAF1 | TAF1 RNA polymerase II, TATA box-binding (TBP)-associated factor | TAFII250 | aa 1397-1467 aa 1520-1590 | P21675 |
| TAF1L | TAF1-like RNA polymerase II, TATA box-binding protein (TBP)-associated factor | TAF(II)210 | aa 1416-1486 aa 1539-1609 | Q8IZX4 |
| TIF1a | Transcriptional intermediary factor 1 | TRIM24, PTC6, RNF82, | aa 932-987 | O15164 |
| TRIM28 | Tripartite motif-containing 28 | KAP1, RNF96, TIF1Î2 | aa 697-801 | Q13263 |
| TRIM33 | Tripartite motif-containing 33 A | PTC7, RFG7, TIF1Î3 | aa 974-1046 | Q9UPN9 |
| TRIM66 | Tripartite motif-containing 66 | TIF1Ì€ | aa 1056-1128 | O15016 |
| WDR9 | WD repeat domain 9 | BRWD1 | aa 1177-1247 aa 1330-1400 | Q9NSI6 |

(continued)

| Protein | Name | Alias | Bromodomain of UniProt SEQ | UniProt SEQ ID |
|---------|------|-------|----------------------------|----------------|
| ZMYND11 | Zinc finger, MYND domain containing 11 | BS69, BRAM1 | aa 168-238 | Q15326 |
| MLL4 | Myeloid/lymphoid or mixed-lineage leukemia 4 | KMT2B, HRX2, KIAA0304, MLL2, TRX2, WBP7 | aa 1395-1509 | Q9UMN6 |

In some embodiments of any of the methods and fusion proteins, the chromatin binding polypeptide is a bromodomain polypeptide. In some embodiments, the bromodomain polypeptide (natively and/or endogenously) comprises a bromodomain module comprising an amino acid sequence characterized by a conserved fold that comprises a left-handed bundle of four $\alpha$ helices ($\alpha$Z, $\alpha$A, $\alpha$B, $\alpha$C), linked by loop regions of variable length (ZA and BC loops). In some embodiments, the bromodomain module of the bromodomain polypeptide comprises a histone $\varepsilon$-N-acetylation of lysine residues (Kac) binding site. In some embodiments, the bromodomain module of the bromodomain polypeptide recognizes Kac by a central deep hydrophobic cavity, where it is anchored by a hydrogen bond to an asparagine residue. In some embodiments, the bromodomain polypeptide comprises at least one bromodomain polypeptide in Table 1. In some embodiments, the bromodomain polypeptide comprises the sequence identified in Table 1 as a UniProt SEQ ID (the UniPort sequences, including canonical sequences, are the sequences as accessed on November 1, 2013 and hereby incorporated by reference in their entirety). In certain embodiments of any of the fusion proteins, the bromodomain polypeptide comprises the amino acid sequence of any one of BRG1, PCAF/KAT2B, BAZ2B, BRD1, BRD8, BRFP1, BRFP3, BRG1, CBP/CREBBP, PCAF/KAT2B, TRIM24, and/or ZMYND8, or a fragment thereof comprising at least one bromodomain module. In certain embodiments, the bromodomain polypeptide comprises the amino acid sequence of any one of BRD2, BRD3, BRD4, BRD9, BRDT, and/or BRG1, or a fragment thereof comprising at least one bromodomain module. In certain embodiments, the bromodomain polypeptide comprises the amino acid sequence of any one of BRG1, BRPF1, CECR2, PCAF, and/or TAF1, or a fragment thereof comprising at least one bromodomain module. In certain embodiments, the bromodomain polypeptide comprises the amino acid sequence of BRD4 and/or BRD9, or a fragment thereof comprising at least one bromodomain module. In certain embodiments, the bromodomain polypeptide comprises a full length bromodomain polypeptide. In some embodiments, the fusion protein comprises a fragment of the bromodomain polypeptide comprising the bromodomain module. In some embodiments, the fusion protein comprises at least about 50, 60, 70, 80, and 90% of the bromodomain polypeptide and includes the bromodomain module.

The fusion protein as described herein comprises a reporter module. Reporter modules are known in the art, and include, but are not limited to, $\beta$-galactosidase (lacZ), chloramphenicol acetyltransferase (cat), $\beta$- glucuronidase (GUS), fluorescent protein, and/or luciferase. In some embodiments of any of the fusion proteins and methods described herein, the reporter module is a reporter protein capable of and/or assembles into multimers ("multimeric reporter protein"). In some embodiments, the multimeric reporter protein is an obligate dimeric protein. In some embodiments, the multimeric reporter protein is an obligate trimeric protein. In some embodiments, the multimeric reporter protein is an obligate tetrameric protein. In some embodiments, the multimeric reporter protein is capable and/or forms protein aggregates. In some embodiments, the multimeric reporter protein is capable and/or forms protein oligomers. In certain embodiments, the reporter module comprises a fluorescent reporter module. The fluorescent reporter module comprises a fluorescent protein (see, *e.g.*, Olenych et al., (2006). Cell Biol. 21.5.1-21.5.34; Nathan et al., Journal of Cell Science 120, 4247-4260 (2007); Day et al., Chem. Soc. Rev., 2009, 38, 2887-2921). In some embodiments, the fluorescent reporter module comprises the amino acid sequence of a protein described in Table 2. In some embodiments, the fluorescent reporter module comprises the amino acid sequence of any one of EGFP, TurboGFP, dsRed2, dsRed-Express2, and/or ZsGreen.

Table 2 Fluorescent Report Modules

| Protein (acronym) | Ex/nm | Em/nm | EC/$10^{-3}$ M$^{-1}$ cm$^{-1}$ | QY | Quaternary structure |
|-------------------|-------|-------|------------------------------------|-----|----------------------|
| AmCyan | 458 | 489 | 44 | 0.24 | Tetramer |
| AQ143 | 595 | 655 | 90 | 0.04 | Tetramer |
| AsRed2 | 576 | 592 | 56.2 | 0.05 | Tetramer |
| CopGFP | 482 | 502 | 70 | 0.6 | Tetramer |
| DsRed | 558 | 583 | 75 | 0.79 | Tetramer |
| DsRed2 | 563 | 582 | 43.8 | 0.55 | Tetramer |

(continued)

| Protein (acronym) | Ex/nm | Em/nm | EC/$10^{-3}$ $M^{-1}$ $cm^{-1}$ | QY | Quaternary structure |
|---|---|---|---|---|---|
| DsRed-Express (T1) | 555 | 584 | 38 | 0.51 | Tetramer |
| DsRed-Express2 | 554 | 586 | 35.6 | 0.42 | Tetramer |
| DsRed-Max | 560 | 589 | 48 | 0.41 | Tetramer |
| dTomato | 554 | 581 | 69 | 0.69 | Dimer |
| EGFP | 484 | 507 | | 0.60 | Weak dimer |
| eqFP611 | 559 | 611 | 78 | 0.45 | Tetramer |
| HcRed1 | 588 | 618 | 20 | 0.015 | Dimer |
| JRed | 584 | 610 | 44 | 0.20 | Dimer |
| Katushka | 588 | 635 | 65 | 0.34 | Dimer |
| Midori-ishi Cyan | 472 | 495 | 27.3 | 0.90 | Dimer |
| PhiYFP | 525 | 537 | 130 | 0.39 | Dimer |
| TurboGFP | 482 | 502 | 70 | 0.53 | Dimer |
| TurboRFP | 553 | 574 | 92 | 0.67 | Dimer |
| ZsGreen | 493 | 505 | 43 | 0.91 | Tetramer |
| ZsYellow | 529 | 539 | 20.2 | 0.42 | Tetramer |

In some embodiments of any of the fusion proteins and methods described herein, the fusion protein comprises a nuclear localization signal (NLS). In some embodiments, the NLS comprises the NLS of a chromatin binding polypeptide. In some embodiments, the NLS comprises the NLS of a bromodomain polypeptide. In certain embodiments, the NLS is the SV40 Large T-antigen NLS or the NLS of nucleoplasmin.

The reporter module can be any position within the fusion protein that allows for detection and does not significantly (e.g., does not) interfere with the interaction of the chromatin binding module with chromatin. In some embodiments of any of the fusion proteins and methods described herein, the chromatin binding module is located 5' of the reporter module. In some embodiments of any of the fusion proteins and methods described herein, the chromatin binding module is located 3' of the reporter module.

In some embodiments of any of the fusion proteins and methods described herein, the fusion protein is capable of multimerizing. In certain embodiments, the fusion protein is capable of forming a dimer, a trimer or a tetramer. In certain embodiments, the fusion protein is capable of forming a dimer. In certain embodiments, the fusion protein is capable of forming a tetramer. In some embodiments, the fusion protein is capable of forming protein aggregates. In some embodiments, the fusion protein is capable of oligomerizing.

A plurality of fusions proteins can associate to form foci, through, e.g., multimerization of proteins in the reporter modules. The foci can then be detected. In certain embodiments, the reporter module comprises a fluorescent reporter module.

In some embodiments of any of the fusion proteins and methods described herein, formation of a foci and/or dot is determined by comparison of the localization of the fusion protein in a control or reference sample. In some embodiments, the localization of the fusion protein in the control or reference sample is the localization of the fusion protein in a cell in the absence of a chromatin binding module inhibiting compound. In some embodiments, treatment of a cell with and/or presence of a chromatin binding module inhibiting compound will result in an increase of greater than about any of 10, 20, 30, 40, 50, 60, 70, 80, 90% in the number of foci in a cell compared to an untreated cell and/or absence of a chromatin binding module inhibiting compound. The size and shape of foci and/or dots can vary depending of the chromatin binding module.

The fusion proteins of the present invention are capable of forming foci, which can be detected and measured using methods known in the art. In some embodiments, the formation of foci indicates that the fusion protein has been disassociated from chromatin and the formation of foci and/or dots. Methods of detecting disassociation from chromatin and nuclear localization of proteins are known in the art. In some embodiments, the method of detection is direct detection. In some embodiments, the method of detection is indirect detection with fluorescent-labeled antibodies against reporter module or an engineered protein epitope tag as part of the fusion protein. Localization of the fusion protein is determined with fluorescent microscopy. In some embodiments, the method of detection is biochemical fractionation according to molecular size followed by Western blotting with antibodies against the fusion protein, reporter module or an engineered protein epitope tag as part of the fusion protein.

In certain embodiments, the chromatin binding module does not comprise a member of the malignant brain tumor (MBT) family of chromatin-interacting transcriptional repressors. In certain embodiments, the chromatin binding module does not comprise full length L3MBTL3. In certain embodiments, the chromatin binding module does not comprise the three MBT domains of L3MBTL3. In certain embodiments, the reporter module does not comprise a GFP.

Further provided herein are chromatin binding module inhibiting compounds identified by a method described herein. In some embodiments, the chromatin binding module inhibiting compound is a small molecule inhibitor. In some embodiments, the chromatin binding module inhibiting compound is a bromodomain module inhibiting compound.

### Polypeptide Variants of Chromatin Binding Modules and Chromatin Binding Polypeptides

Variants of the chromatin binding modules and chromatin binding polypeptides are useful in the methods described herein and for use in the fusion proteins described herein. Conservative substitutions of polypeptides are shown in Table 3 under the heading of "preferred substitutions". If such substitutions result in a change in biological activity, then more substantial changes, denominated "exemplary substitutions" in Table 3, or as further described below in reference to amino acid classes, may be introduced and the products screened.

Table 3.

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in the biological properties of the polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Non-conservative substitutions will entail exchanging a member of one of these classes for another class. Amino acids may be grouped according to common side-chain properties:

(1) hydrophobic: Norleucine, Met, Ala, Val, Leu, Ile;

(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe;
(7) large hydrophobic: Norleucine, Met, Val, Leu, Ile.

In further embodiments, polypeptides of the may comprise one or more non-naturally occurring or modified amino acids. A "non-naturally occurring amino acid residue" refers to a residue, other than those naturally occurring amino acid residues listed above, which is able to covalently bind adjacent amino acid residues(s) in a polypeptide chain. Non-natural amino acids include, but are not limited to homo-lysine, homo-arginine, homo-serine, azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2-aminoheptanoic acid, 2aminoisobutyric acid, 3-aminoisbutyric acid, 2-aminopimelic acid, tertiary-butylglycine, 2,4-diaminoisobutyric acid, desmosine, 2,2'-diaminopimelic acid, 2,3-diaminopropionic acid, N-ethylglycine, N-ethylasparagine, homoproline, hydroxylysine, allo-hydroxylysine, 3-hydroxyproline, 4-hydroxyproline, isodesmosine, allo-isoleucine, N-methylalanine, N-methylglycine, N-methylisoleucine, N-methylpentylglycine, N-methylvaline, naphthalanine, norvaline, norleucine, ornithine, citrulline, pentylglycine, pipecolic acid and thioproline. Modified amino acids include natural and non-natural amino acids which are chemically blocked, reversibly or irreversibly, or modified on their N-terminal amino group or their side chain groups, as for example, N-methylated D and L amino acids, side chain functional groups that are chemically modified to another functional group. For example, modified amino acids include methionine sulfoxide; methionine sulfone; aspartic acid- (beta-methyl ester), a modified amino acid of aspartic acid; N-ethylglycine, a modified amino acid of glycine; or alanine carboxamide and a modified amino acid of alanine. Additional non-natural and modified amino acids, and methods of incorporating them into proteins and peptides, are known in the art (*see*, *e.g.*, Sandberg et al., (1998) J. Med. Chem. 41: 2481-91; Xie and Schultz (2005) Curr. Opin. Chem. Biol. 9: 548-554; Hodgson and Sanderson (2004) Chem. Soc. Rev. 33: 422-430.

Variants of the polypeptide comprising the chromatin binding module can also be made based on information known in the art, without substantially affecting the activity of chromatin binding module. For example, polypeptide comprising the chromatin binding module and/or chromatin binding module variants can have at least one amino acid residue in the polypeptide comprising the chromatin binding module and/or chromatin binding module replaced by a different residue.

A convenient way for generating such substitutional variants involves phage display. The phage-displayed variants are then screened for their biological activity (e.g. binding affinity) as herein disclosed. In order to identify candidate regions of the polypeptide comprising the chromatin binding module and/or chromatin binding module for modification, alanine scanning mutagenesis can be performed to identify hypervariable region residues contributing significantly to chromatin binding. Once such variants are generated, the panel of variants is subjected to screening as described herein and antibodies with superior properties in one or more relevant assays may be selected for further development.

### Nucleic Acids, Expression Cassettes, Vectors, and Cells Used in the Methods Described Herein

Provided herein are nucleic acids encoding the fusion proteins described herein, and expression cassettes, vectors comprising the nucleic acids comprising the fusion proteins described herein. Provided herein are isolated and/or substantially purified fusion proteins and nucleic acids (*e.g.*, DNA or RNA) encoding the fusion proteins described herein. Further provided are vectors and expression cassettes comprising nucleic acids encoding the fusion proteins described herein.

Polynucleotide sequences encoding the polypeptide comprising the chromatin binding module and/or chromatin binding module described herein can be obtained using standard synthetic and/or recombinant techniques. Desired polynucleotide sequences may be isolated and sequenced from appropriate source cells. Source cells for polypeptide comprising the chromatin binding module and/or chromatin binding module would include polypeptide comprising the chromatin binding module and/or chromatin binding module producing cells such as hybridoma cells. Alternatively, polynucleotides can be synthesized using nucleotide synthesizer or PCR techniques. Once obtained, sequences encoding polypeptide comprising the bromodomain and/or bromodomain are inserted into a recombinant vector capable of replicating and expressing heterologous polynucleotides in a host cell. Many vectors that are available and known in the art can be used for the purpose of the present disclosure. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication (in particular when the vector is inserted into a prokaryotic cell), a selection marker gene, a promoter, a ribosome binding site (RBS), a signal sequence, the heterologous nucleic acid insert and a transcription termination sequence.

Nucleic acid molecules encoding amino acid sequence variants of the polypeptide comprising a chromatin binding module and/or chromatin binding module are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of polypeptide comprising the chromatin binding module and/or chromatin binding module.

Also provided is a vector containing an expression cassette comprising a promoter operably linked to a target sequence. "Expression cassette" as used herein means a nucleic acid sequence capable of directing expression of a particular nucleotide sequence in an appropriate host cell, which includes a promoter operably linked to the nucleotide sequence of interest that may be operably linked to termination signals. The coding region usually codes for a functional RNA of interest, for example an RNAi molecule. The expression cassette including the nucleotide sequence of interest may be chimeric. Nucleic acids can be engineered into a vector using standard techniques, such as those described in Sambrook and Russell, Molecular Cloning: A Laboratory Manual, Cold Spring Harbor Laboratory Press Cold Spring Harbor, NY (2001).

In general, plasmid vectors containing replicon and control sequences which are derived from a species compatible with the host cell are used in connection with these hosts. The vector ordinarily carries a replication site, as well as marking sequences which are capable of providing phenotypic selection in transformed cells. In addition, phage vectors containing replicon and control sequences that are compatible with the host microorganism can be used as transforming vectors in connection with these hosts.

Either constitutive or inducible promoters can be used in the present invention, in accordance with the needs of a particular situation, which can be ascertained by one skilled in the art. A large number of promoters recognized by a variety of potential host cells are well known. The selected promoter can be operably linked to cistron DNA encoding a polypeptide described herein by removing the promoter from the source DNA via restriction enzyme digestion and inserting the isolated promoter sequence into the vector of choice. Both the native promoter sequence and many heterologous promoters may be used to direct amplification and/or expression of the fusion proteins described herein. However, heterologous promoters are preferred, as they generally permit greater transcription and higher yields of expressed target gene as compared to the native target polypeptide promoter.

In some embodiments, each cistron within a recombinant vector comprises a secretion signal sequence component and/or nuclear localization signal that directs translocation of the expressed polypeptides across a membrane. In general, the signal sequence may be a component of the vector, or it may be a part of the target polypeptide DNA that is inserted into the vector. The signal sequence selected for the purpose of this invention should be one that is recognized and processed (*i.e.* cleaved by a signal peptidase) by the host cell.

Provided herein are also cells comprising the expression cassette and/or vector comprising the nucleic acids comprising the fusion proteins described herein. Provided herein are cells comprising a fusion protein described herein. In some embodiments, the cell is a eukaryotic cell. In some embodiments, the cell is a mammalian cell. In certain embodiments, the cell is a CHO-K1, COS-7, HEK293, HEK293T, HEK293FT, HeLa, MDCK, and/or U2OS cell. In certain embodiments, the cell is a COS-7, HeLa, and/or U2OS cell.

Host cells are transformed or transfected with the above-described expression vectors or transduced with virus packaged with the above-describe expression vectors and cultured in conventional nutrient media modified as appropriate for inducing promoters, selecting transformants, or amplifying the genes encoding the desired sequences. Transfection refers to the taking up of an expression vector by a host cell whether or not any coding sequences are in fact expressed. Successful transfection is generally recognized when any indication of the operation of this vector occurs within the host cell.

If an inducible promoter is used in the expression vector, protein expression is induced under conditions suitable for the activation of the promoter. A variety of other inducers may be used, according to the vector construct employed, as is known in the art. Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

Cells may be removed from the culture and the culture supernatant being filtered and concentrated for further purification of the proteins produced. The expressed polypeptides can be further isolated and identified using commonly known methods such as fractionation on immunoaffinity or ion-exchange columns; ethanol precipitation; reverse phase HPLC; chromatography on silica or on a cation exchange resin such as DEAE; chromatofocusing; SDS-PAGE; ammonium sulfate precipitation; gel filtration using, for example, Sephadex G-75; hydrophobic affinity resins, ligand affinity using a suitable antigen immobilized on a matrix and Western blot assay.

***Therapeutic/Prophylactic Methods and/or Uses***

Chromatin binding module inhibiting compounds identified by any of the methods described herein may be useful in therapeutic methods.

In one aspect, a chromatin binding module inhibiting compound for use as a medicament is provided. In further aspects, a chromatin binding module inhibiting compound for use in a method of treating cancer is provided. Also provided is a chromatin binding module inhibiting compound for use in a method of treating in an individual comprising administering to the individual an effective of the chromatin binding module inhibiting compound to treat cancer. In certain embodiments, a chromatin binding module inhibiting compound for use in a method of treatment is provided. In certain embodiments, provided are chromatin binding module inhibiting compound for use in a method of treating an individual having cancer comprising administering to the individual an effective amount of the chromatin binding module inhibiting compound. In one such embodiment, the method further comprises administering to the individual an effective amount of at least one additional therapeutic agent, *e.g.,* as described below. Also provided is a chromatin binding module inhibiting compound for use in treating cancer. Also provided is a chromatin binding module inhibiting compound for use in a method treating cancer in an individual comprising administering to the individual an effective amount of the chromatin binding module inhibiting compound to treat cancer. Also provided is a chromatin binding module inhibiting compound for use in treating cancer. Also provided is a chromatin binding module inhibiting compound for use in a method of inhibiting cell proliferation in an individual comprising administering to the individual an effective amount of the chromatin binding module inhibiting compound to inhibit cell proliferation. In some embodiments, chromatin binding module inhibiting compound is a bromodomain module inhibiting compound. An "individual" according to any of the above embodiments is preferably a human.

In a further aspect, provided herein are pharmaceutical formulations comprising any of the chromatin binding module inhibiting compounds, *e.g.*, for use in any of the above therapeutic methods. In one embodiment, a pharmaceutical formulation comprises any of the chromatin binding module inhibiting compounds and a pharmaceutically acceptable carrier. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutralactive hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

In some embodiments, a pharmaceutical formulation comprises a chromatin binding module inhibiting compound and at least one additional therapeutic agent, *e.g.,* as described below.

The chromatin binding module inhibiting compound can be used either alone or in combination with other agents in a therapy. For instance, a chromatin binding module inhibiting compound may be co-administered with at least one additional therapeutic agent.

Such combination therapies noted above encompass combined administration (where two or more therapeutic agents are included in the same or separate formulations), and separate administration, in which case, administration of the chromatin binding module inhibiting compound described herein can occur prior to, simultaneously, and/or following, administration of the additional therapeutic agent.

A chromatin binding module inhibiting compound described herein (and any additional therapeutic agent) can be administered by any suitable means, including parenteral, intrapulmonary, and intranasal, and, if desired for local treatment, intralesional administration, topical administration, or intraocular administration. Parenteral infusions include intramuscular, intravenous, intraarterial, intraperitoneal, or subcutaneous administration. Dosing can be by any suitable route, *e.g.* by injections, such as intravenous or subcutaneous injections, depending in part on whether the administration is brief or chronic. Various dosing schedules including but not limited to single or multiple administrations over various time-points, bolus administration, and pulse infusion are contemplated herein.

Examples of bromodomain-mediated disorders include cancers, including, but not limited, to acoustic neuroma, acute leukemia, acute lymphocytic leukemia, acute myelocytic leukemia (monocytic, myeloblastic, adenocarcinoma, angiosarcoma, astrocytoma, myelomonocytic and promyelocytic), acute t-cellleukemia, basal cell carcinoma, bile duct carcinoma, bladder cancer, brain cancer, breast cancer, bronchogenic carcinoma, cervical cancer, chondrosarcoma, chordoma, choriocarcinoma, chronic leukemia, chronic lymphocytic leukemia, chronic myelocytic (granulocytic) leukemia, chronic myelogenous leukemia, colon cancer, colorectal cancer, craniopharyngioma, cystadenocarcinoma, diffuse large B-cell lymphoma, dysproliferative changes (dysplasias and metaplasias), embryonal carcinoma, endometrial cancer, endotheliosarcoma, ependymoma, epithelial carcinoma, erythroleukemia, esophageal cancer, estrogen-receptor positive breast cancer, essential thrombocythemia, Ewing's tumor, fibrosarcoma, follicular lymphoma, germ cell testicular cancer, glioma, glioblastoma, gliosarcoma, heavy chain disease, hemangioblastoma, hepatoma, hepatocellular cancer, hormone insensitive prostate cancer, leiomyosarcoma, leukemia, liposarcoma, lung cancer, lymphagioendotheliosarcoma, lymphangiosarcoma, lymphoblastic leukemia, lymphoma (Hodgkin's and non-Hodgkin's), malignancies and hyperproliferative disorders of the bladder, breast, colon, lung, ovaries, pancreas, prostate, skin and uterus, lymphoid malignancies ofT-cell or B-cell origin, leukemia, lymphoma, medullary carcinoma, medulloblastoma, melanoma, meningioma, mesothelioma, multiple myeloma, myelogenous leukemia, myeloma, myxosarcoma, neuroblastoma, NUT midline carcinoma (NMC), non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom's macroglobulinemia, testicular tumors, uterine cancer and Wilms' tumor.

In certain embodiments of any of the methods, the cancer is lung cancer, breast cancer, pancreatic cancer, colorectal cancer, and/or melanoma. In certain embodiments, the cancer is lung. In certain embodiments, the lung cancer is NSCLC. In certain embodiments, the cancer is breast cancer. In certain embodiments, the cancer is melanoma.

### *Articles of Manufacture/Kits*

Provided herein are also articles of manufacture containing materials useful for identifying chromatin binding module inhibiting compounds comprising the fusion proteins, nucleic acids, expression cassettes, vectors, and/or cells described herein. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for identifying chromatin binding module inhibiting compounds.

The label or package insert indicates that the composition is used for identifying chromatin binding module inhibiting compounds. Moreover, the article of manufacture may comprise (a) a first container with a composition contained therein, wherein the composition comprises a fusion protein, nucleic acid, expression cassette, vector, and/or cell described herein described herein; and (b) a second container with a composition contained therein, wherein the composition comprises an additional reagent. The article of manufacture in this embodiment of the disclosure may further comprise a package insert indicating that the compositions can be used for identifying chromatin binding module inhibiting compounds.

The following examples are included to demonstrate preferred embodiments of the present invention. It should be appreciated by those of skill in the art that the techniques disclosed in the examples that follow represent techniques discovered by the inventors to function well in the practice of the invention, and thus can be considered to constitute preferred modes for its practice. However, the invention is not limited by the examples.

### EXAMPLES

The following are examples of fusion proteins and methods of using the fusion proteins of the invention. It is understood that various other embodiments may be practiced, given the general description provided herein.

### Example 1

To understand the localization changes of fluorescent tags (FPs) on the FP-tagged bromodomain-containing proteins in the nucleus upon release from the chromatin by bromodomain inhibiting compounds, certain fusion configurations of FP-BRD4 resulted in inhibitor-dependent relocalization of FP-tagged proteins and foci formation in the nucleus.

### Methods

Fluorescent protein (FP) tags (dsRed2, dsRed-Express2, EGFP, Emerald GFP, mCherry, mOrange2, TurboGFP, or ZsGreen) were cloned in-frame upstream of the human BRD4 coding DNA sequence (amino acid residues 1-719 of the BRD4 isoform short; NCBI NP _055114.1) in a lentiviral vector, which expresses Tet-On 3G transcription factor under the control of human EF1 promoter. The expression of the FP-BRD4 protein, which was downstream of the inducible TRE3G promoter, could be induced by doxycycline. Lentivirus generated with these expression plasmid constructs was used to infect U2OS osteosarcoma cells (ATCC, HTB-96) to establish stable cells under 15 μg/ml Blasticidin selection. Stable cells were plated in Nunc™ Lab-Tek™ II chamber slides for confocal microscopy or 96-well plates for high-content microscopy. Cells were treated 2 μg/ml doxycycline for 16 hours before incubation with either DMSO or BRD4 inhibitors for 2-4 hours prior to imaging. Zeiss LSM510 inverted confocal microscope or LSM780 confocal microscope with heated stage was used to examine the localization of FP-tagged BRD4 proteins. Acquired images were analyzed with the Zeiss ZEN software. For high-content microscopy, compound-treated cells were fixed with 4% formaldehyde for 15 minutes and washed twice with PBS buffer. Images were acquired on ImageXpress Micro (Molecular Devices) and analyzed with MetaXpress® (Molecular Devices).

## Results

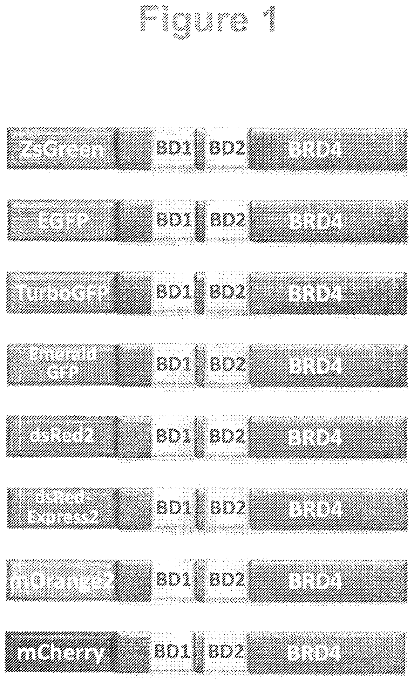

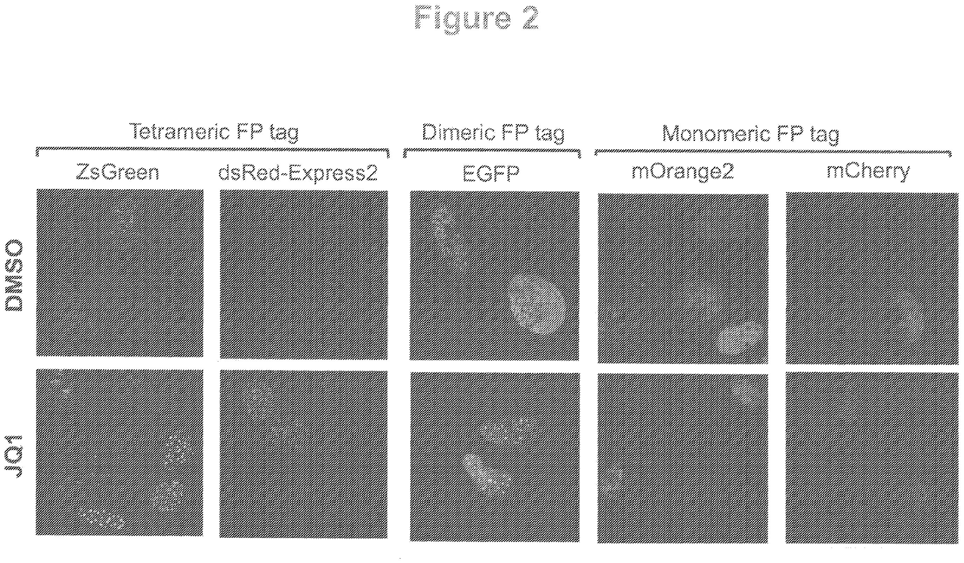

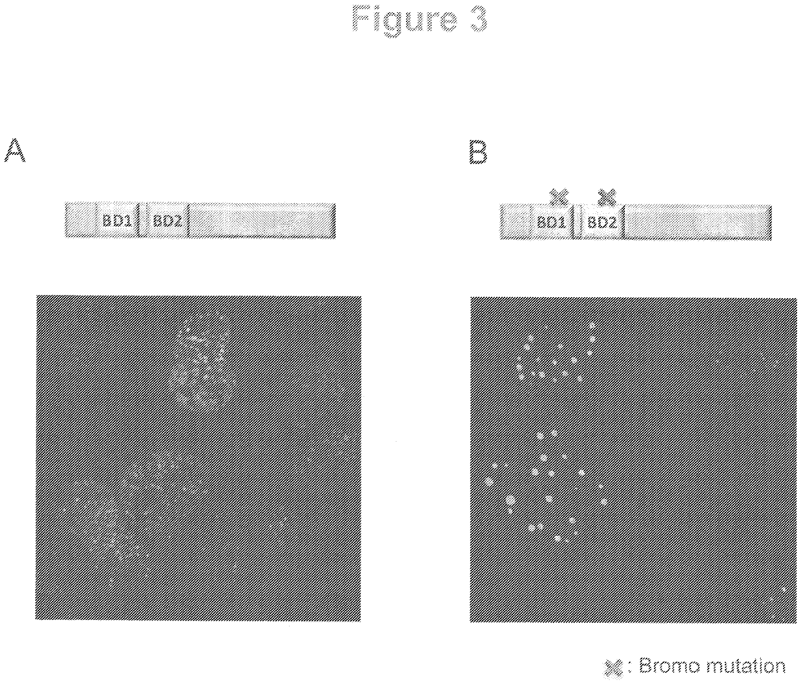

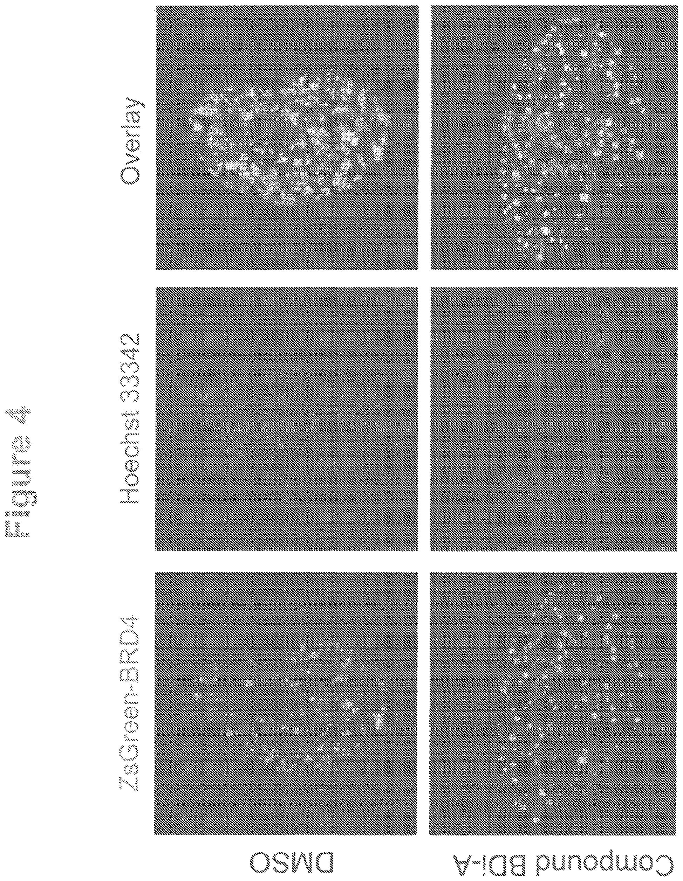

U2OS cell lines stably carrying inducible BRD4 tagged with different fluorescent proteins (FPs) were generated. The N-terminal FP tags included dsRed2, dsRed-Express2, EGFP, Emerald GFP, mCherry, mOrange2, TurboGFP, or ZsGreen (Figure 1). FP-BRD4 expression was induced by doxycycline and cells were treated with either vehicle (DMSO) or BRD4 bromodomain inhibitor JQ1 for 4 hours before live-cell imaging with a confocal microscope. JQ1 (CAS # 126524-70-4) is a compound that inhibits bromodomains of BRD2, BRD3, BRD4 and BRDT. FP-tagged BRD4 proteins bound to chromatin and distributed diffusely in the nucleus when treated with DMSO (Figure 2). Interestingly, JQ1 treatment resulted in protein relocalization and formation of fluorescent dots/foci of EGFP-BRD4, ZsGreen-BRD4, dsRed-Express2, dsRed2-BRD4 and TurboGFP-BRD4. However, inhibition of BRD4 bromodomains of mOrange2-BRD4, mCherry-BRD4 and Emerald-GFP-BRD4 by JQ1 did not change the localization of FP-tagged proteins in the nucleus. These findings showed that fluorescent BRD4 fusion proteins aggregated and formed dots/foci in response to bromodomain inhibitors when BRD4 was tagged with fluorescent proteins that have tendency to form multimers (e.g., dimers or tetramers). Importantly, mutant ZsGreen-BRD4 proteins with point mutations in both bromodomains, which cannot bind to chromatin and therefore mimics the effect of bromodomain inhibitors, were localized to dots/foci in the nucleus even in the absence of bromodomain inhibitors (Figure 3B). Wild type BRD4 fusion proteins showed diffuse localization under the same condition (Figure 3A). To further analyze bromodomain inhibiting compounds prevention of bromodomain binding to the chromatin, chromatin was marked with DNA-binding dye Hoechst 33342. As shown in Figure 4, there was significant colocalization of ZsGreen-BRD4 protein and the chromatin. On the other hand, treatment with bromodomain inhibiting compound BDi-A resulted in fluorescent dots of ZsGreen-BRD4, and those dots did not overlap with the chromatin (Figure 4). Taken together, these results demonstrated that FP-BRD4 proteins relocalized to form dots/foci when the binding activity of bromodomain was inhibited by bromodomain inhibiting compounds or eliminated by point mutation within the bromodomain.

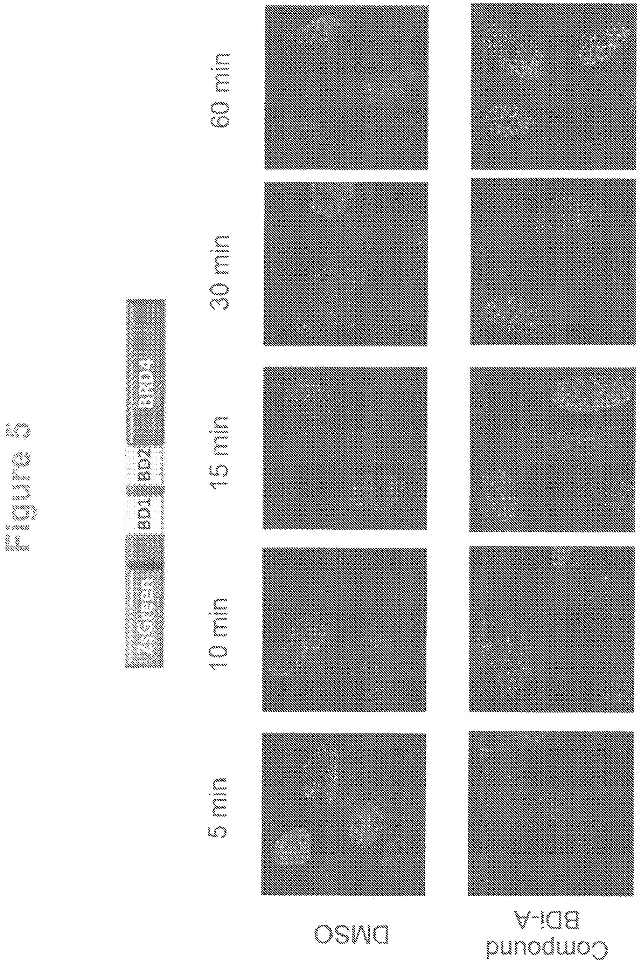

To investigate the kinetics of dot/foci formation, stable cells expressing ZsGreen-BRD4 were treated with DMSO or compound BDi-A (a bromodomain inhibitor of BRD4) for 5 min, 10 min, 15 min, 30 min or 60 min followed by fixation and confocal microscopy. Relocalization of the ZsGreen-BRD4 protein was noticed at the 10-min time point, and dot/foci formation was dramatic by 30 min (Figure 5). These results showed a fast kinetic of ZsGreen-BRD4 relocalization, and the effect of bromodomain inhibitors could be monitored in real-time by following the fluorescent foci formation with microscopy.

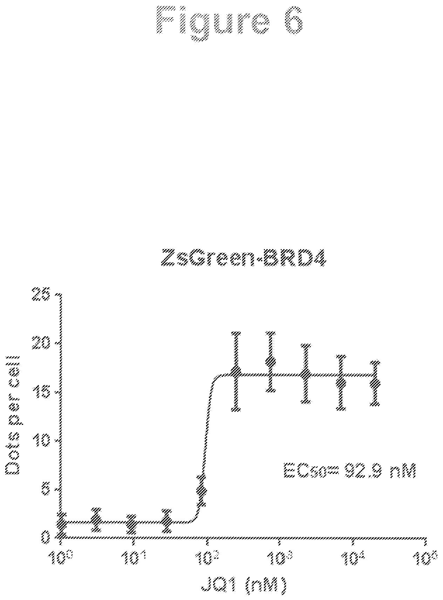

To quantify the effect of BRD4 inhibitors on ZsGreen-BRD4 relocalization, stable cells were seed in a 96-well plate and incubated with doxycycline to induce the expression of ZsGreen-BRD4. Cells were treated with different concentrations of JQ1 for 4 hours prior to fixation. Images were then acquired with a high-content microscope and analyzed with MetaXpress to determine the number of fluorescent dots per cells. The derived data were analyzed in GraphPad Prism to calculate the $EC_{50}$ value. As expected, more ZsGreen-BRD4 dots were observed at higher JQ1 concentrations and gradually decreased at lower JQ1 concentrations. The calculated $EC_{50}$ of JQ1 (CAS # 1268524-70-4) is 93 nM in this BRD4 relocalization assay, called 'Dot Assay' (Figure 6). These results showed that relocalization/dot formation was a titratable phenotype and could be used to determine the potency of bromodomain inhibitors in cellular settings.

## Discussion

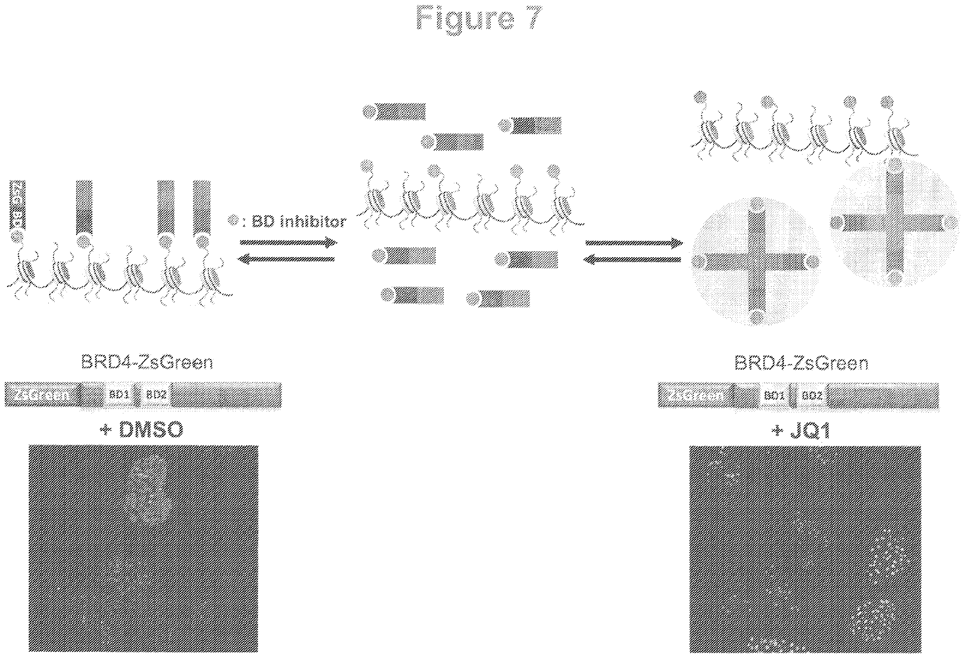

Bromodomain proteins interact with chromatin and have the ability to bind histone tails with various posttranslational modifications. One potential, but non-limiting model, is depicted in Figure 7. As shown herein, BRD4 bromodomain inhibiting compounds and mutations in the bromodomain of BRD4 resulted in the release of BRD4 protein from the chromatin. When BRD4 was tagged with a FP that had the ability to form multimers (e.g., dimers or tetramers), the affinity of FP tags promoted the FP-tagged BRD4 to aggregate and formed dots/foci in the nucleus. Further, as shown herein, BRD4 tagged with monomeric FPs (mOragne2 and mCherry) did not show inhibitor-dependent dots/foci formation (Figure 2). In addition, mutation of the BRD4 bromodomains that abolished the binding ability results in 'dot' phenotype even in the absence of BRD4 bromodomain inhibitors (Figure 3). ZsGreen-BRD4 relocalization, aggregation and dots/foci formation in response to inhibitor were due to the inhibition of bromodomain binding affinity. The FP tags served multiple purposes in this system including promoting aggregation and foci formation of the tagged bromodomain protein and as a detection method.

Example 2

A fusion protein containing full-length ZsGreen and BRD2 was expressed in cells to analyze localization change in response BRD2 bromodomain inhibiting compounds.

Methods

A fluorescent protein (ZsGreen) was cloned in-frame upstream of the human BRD2 coding DNA sequence (NP_001106653.1) in a lentiviral vector, which expresses Tet-On 3G transcription factor under the control of human EF1 promoter. The expression of the ZsGreen-BRD2 coding region, which was downstream of the inducible TRE3G promoter, could be induced by doxycycline. Lentivirus generated with these expression plasmid constructs was used to infect U2OS osteosarcoma cells (ATCC, HTB-96) to establish stable cells under 15 μg/ml Blasticidin selection. Stable cells were plated in Nunc™ Lab-Tek™ II chamber slides for confocal microscopy. Cells were treated 2 μg/ml doxycycline for 16 hours before incubation with either DMSO or BRD2 inhibitors for 4 hours prior to imaging. Zeiss LSM510 inverted confocal microscope or LSM780 confocal microscope with heated stage was used to examine subnuclear localization of FP-tagged BRD2. Acquired images were analyzed with the Zeiss ZEN software.

Results and Discussion

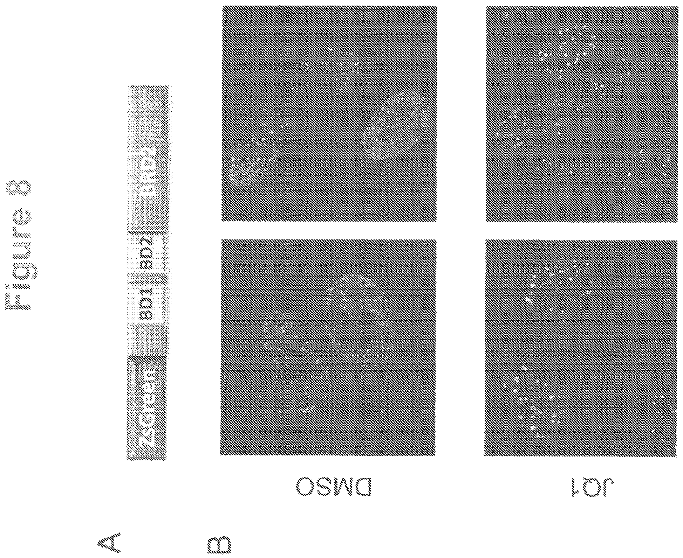

U2OS cell lines stably carrying inducible N-terminally ZsGreen-tagged full-length BRD2 (Figure 8A) were generated through lentiviral infection and Blasticidin selection. To test whether ZsGreen-BRD2 protein forms dots/foci after being released from the chromatin by bromodomain inhibitors, stable U2OS/ZsGreen-BRD2 cells were induced with doxycycline and treated with either vehicle (DMSO) or bromodomain inhibitor JQ1 (CAS # 126524-70-4) at 10 μM for 4 hours before live-cell imaging with a confocal microscope. JQ1 treatment resulted in ZsGreen-BRD2 protein relocalization and the formation of dots/foci in the nucleus (Figure 8B). This inhibitor-dependent foci formation phenotype was similar to the aggregation phenotype observed in the case of BRD4 fused to FP tags that form dimers or multimers (Figure 2).

Example 3

A fusion protein containing full-length ZsGreen and BRD3 was expressed in cells to analyze localization change in response to BRD3 bromodomain inhibiting compounds.

Methods

A fluorescent protein (ZsGreen) was cloned in-frame upstream of the human BRD3 coding DNA sequence (NP_031397.1) in a lentiviral vector, which expresses Tet-On 3G transcription factor under the control of human EF1 promoter. The expression of the ZsGreen-BRD3 coding region, which was downstream of the inducible TRE3G promoter, could be induced by doxycycline. Lentivirus generated with these expression plasmid constructs was used to infect U2OS osteosarcoma cells (ATCC, HTB-96) to establish stable cells under 15 μg/ml Blasticidin selection. Stable cells were plated in Nunc™ Lab-Tek™ II chamber slides for confocal microscopy. Cells were treated 2 μg/ml doxycycline for 16 hours before incubation with either DMSO or BRD3 inhibitors for 4 hours prior to imaging. Zeiss LSM510 inverted confocal microscope or LSM780 confocal microscope with heated stage was used to examine subnuclear localization of FP-tagged BRD3. Acquired images were analyzed with the Zeiss ZEN software.

Results and Discussion

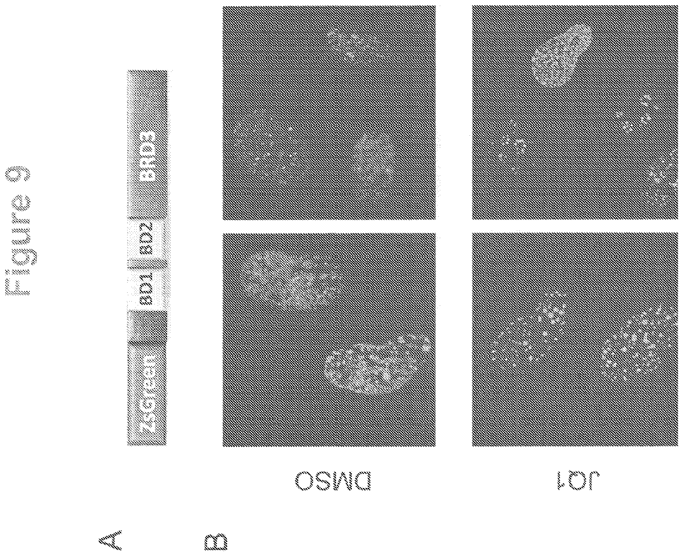

U2OS cell lines stably carrying inducible N-terminally ZsGreen-tagged full-length BRD3 (Figure 9A) were generated through lentiviral infection and Blasticidin selection. To test whether ZsGreen-BRD3 protein forms dots/foci after released from the chromatin by bromodomain inhibitors, stable U2OS/ZsGreen-BRD3 cells were treated with doxycycline and incubated with either vehicle (DMSO) or bromodomain inhibitor JQ1 (CAS #1268524-70-4) at 10 μM for 4 hours before live-cell imaging. Similar to the findings made with ZsGreen-BRD2 and ZsGreen-BRD4, ZsGreen-BRD3 protein formed fluorescent dots/foci in response to the BRD3 bromodomain inhibiting compound JQ1 (Figure 9B).

**Example 4**

Experiments in this example were designed and conducted to establish a method to determine the cellular effect of compounds that inhibit BRD9 bromodomain. Fusion proteins containing full-length BRD9 and fluorescent proteins were expressed in cells to analyze localization change and foci formation caused by bromodomain inhibitors.

**Methods**

A fluorescent protein (ZsGreen or mVenus) was cloned in-frame downstream of the human BRD9 coding DNA sequence (NCBI NP_076413.3) in a lentiviral vector, which expresses Tet-On 3G transcription factor under the control of human EF1 promoter. The expression of the BRD9-ZsGreen and BRD9-ZsGreen coding regions, which were located downstream of the inducible TRE3G promoter, could be induced by doxycycline. Lentivirus generated with these expression plasmid constructs was used to infect U2OS osteosarcoma cells (ATCC, HTB-96) to establish stable cells under 15 µg/ml Blasticidin selection. Stable cells were plated in Nunc™ Lab-Tek™ II chamber slides for confocal microscopy. Cells were treated 2 µg/ml doxycycline for 16 hours before incubation with either DMSO or BRD9 inhibitors (BDi-B, BDi-C and BDi-D) for 4 hours prior to imaging. Zeiss LSM510 inverted confocal microscope or LSM780 confocal microscope with heated stage was used to examine localization of FP-tagged BRD9 in the nucleus. Acquired images were analyzed with the Zeiss ZEN software.

**Results and Discussion**

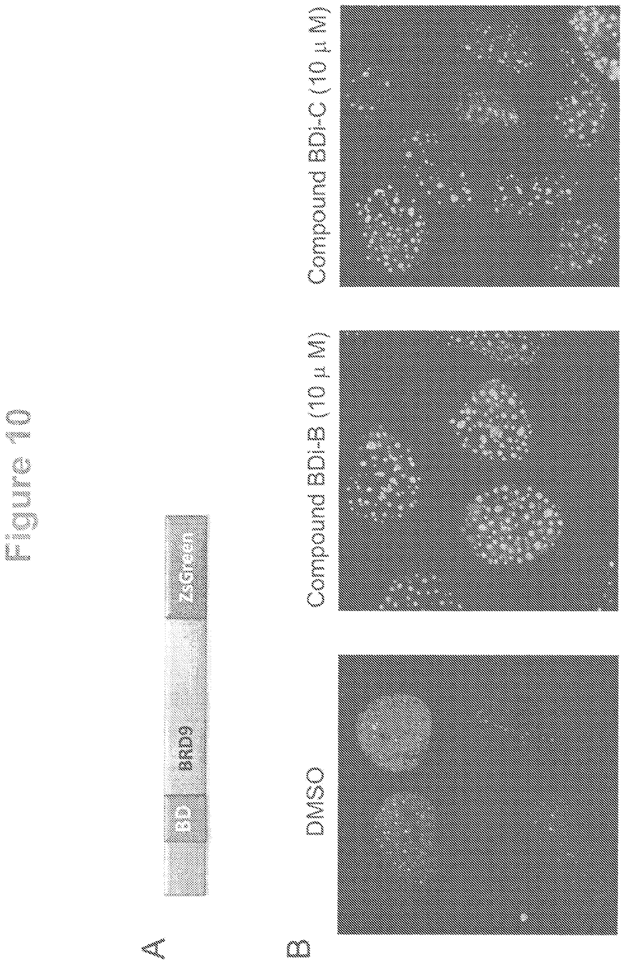

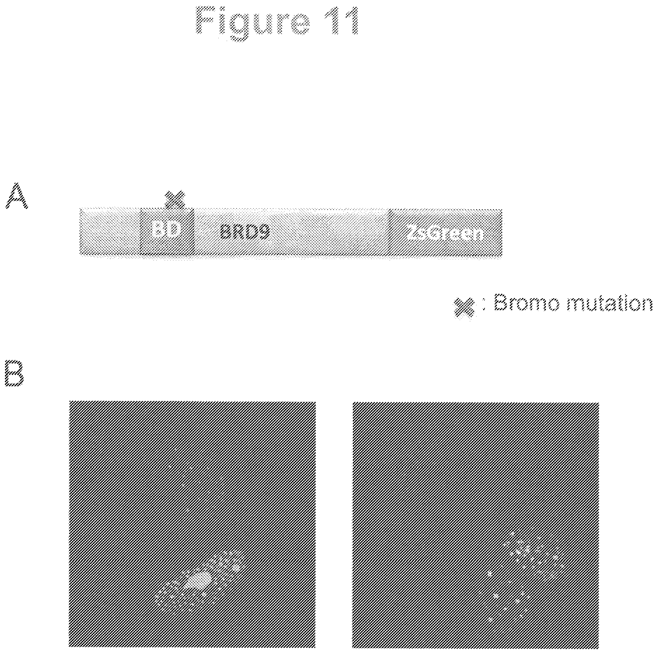

U2OS cell lines stably carrying inducible full length BRD9 with C-terminally ZsGreen tag (Figure 10A) were generated through lentiviral infection and Blasticidin selection. To further investigate the effect of bromodomain inhibition on BRD9-ZsGreen localization, expression of the fluorescent fusion protein was induced by doxycycline and cells were treated with either vehicle (DMSO) or BRD9 bromodomain inhibitors (compounds BDi-C or BDi-D) at 10 µM for 4 hours before live-cell imaging with a confocal microscope. Localization of BRD9-ZsGreen changed from diffuse distribution to fluorescent dots/foci in response to both BRD9 inhibitors (Figure 10B). These results indicated that BRD9-ZsGreen was displaced from the chromatin by the BRD9 bromodomain inhibitors and then formed fluorescent dots/foci. Further, when a point mutation (N216Y) was introduced in the bromodomain of BRD9 to abolish its binding activity (Figure 11A), the mutant BRD9-ZsGreen lacking the binding affinity toward chromatin formed fluorescent dots even in the absence of chromatin inhibiting compounds (Figure 11B). Taken together, the 'dot' phenotype of this bromodomain mutant protein also indicated the 'dot' phenotype of the wild type BRD9-ZsGreen with compound treatment was due to the functional disruption of the BRD9 bromodomain.

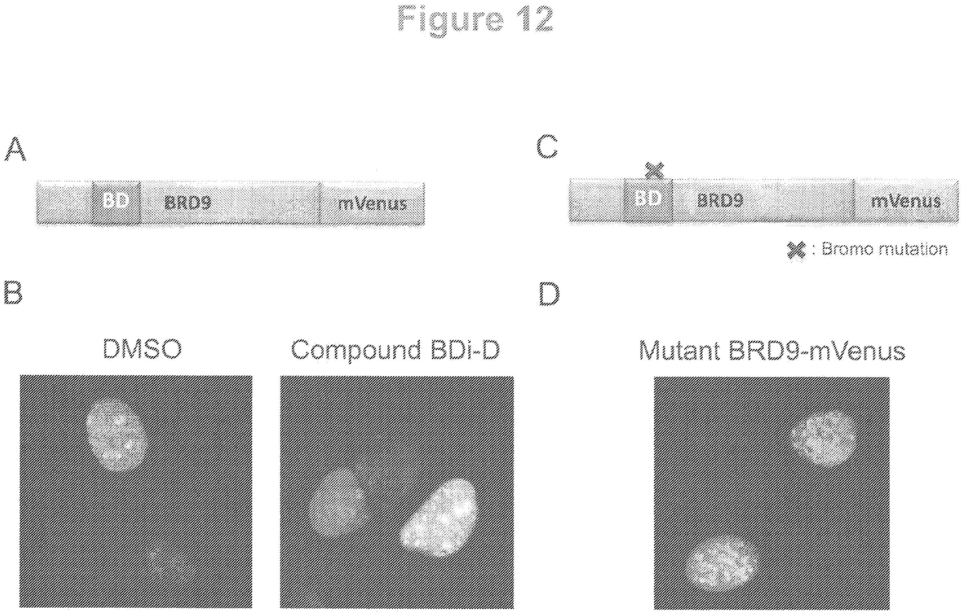

To investigate the contribution of the FP tags on the 'dot/foci' phenotype, BRD9 was fused to a monomeric FP, mVenus, which did not promote protein aggregation (Figure 12A). As expected, there was no clear localization difference for BRD9-mVenus with either DMSO or BRD9 inhibitor (compound BDi-D) and the fluorescent signal distributed evenly in the nucleus (Figure 12B). A bromodomain mutant allele (N216Y) of the mVenus-tagged BRD9 was generated to further confirm the findings with bromodomain inhibitor (Figure 12C). In contrast to the mutant BRD9-ZsGreen (Figure 11B), the mutant BRD9-mVenus was located in the nucleus diffusely even though it cannot bind to the chromatin (Figure 12D). These results again demonstrated the effect of fluorescent proteins on the localization of tagged bromodomain proteins. ZsGreen protein had the ability to form tetramers, whereas mVenus was present as monomeric protein. When the FP tags had the tendency to oligomerize, tagged bromodomain proteins released from the chromatin by inhibitors could aggregate and formed fluorescent dots/foci. If the tags were not capable of forming oligomers (e.g., mVenus), the tagged bromodomain proteins remained diffuse distribution even though they did not bind to the chromatin. Note that chromatin distribution in most of cells was diffuse (Figure 4) and microscopy did not have the resolution to differentiate the diffuse localization of BRD9-mVenus between bound and unbound states.

**Example 5**

The use of bromodomain modules, in the absence of other regions of the bromodomain-containing protein, together with a reporter module (here fluorescent protein tags) to establish assay methods and determine inhibitor activity in cells was investigated.

**Methods**

Three copies of nuclear localization sequence (DPKKKRKV) (SEQ ID NO:1) were fused to the N-terminal of the CECR2 bromodomain coding DNA sequence (amino acid residues 424 -538 of NCBI NP_113601.2) followed by the ZsGreen coding sequence. This fusion expression construct was cloned into a lentiviral vector, which expresses Tet-On 3G transcription factor under the control of human EF1 promoter. The expression of the NLS-CECR2.BD-ZsGreen, controlled by the inducible TRE3G promoter, was induced by doxycycline. Lentivirus generated with these expression plasmid constructs was used to infect U2OS osteosarcoma cells (ATCC, HTB-96) to establish stable cells under 15 μg/ml Blasticidin selection. Stable cells were plated in 6-well or 96-well plates for microscopic analysis. Cells were treated 2 μg/ml doxycycline for 16 hours before incubation with either DMSO or CECR2 inhibitor for 4 hours prior to fixation with 4% formaldehyde. Nikon inverted fluorescent microscope (Eclipse TS100) or ImageXpress Micro (Molecular Devices) was used to examine localization of NLS-CECR2.BD-ZsGreen in the nucleus.

The coding sequence of the fusion protein was amplified from the lentiviral vector described above and ligated into a pET-based bacterial expression vector. The vector expressing the parent ZsGreen protein lacking the CECR2 fusion was purchased from Clontech. Each vector was transformed into BL-21 pLysS Rosetta cells (EMD Millipore), and single colonies were selected by plating on carbenicillin. Cultures were grown in LB broth, and expression of protein was induced by addition of IPTG (at A600 = 0.4-0.6). Cells were harvested after 2.5 h expression and stored at -80°C overnight. Cells were resuspended in 50 mM Tris, pH 7.5, 300 mM NaCl, 1 mM EDTA, and 1 mM DTT and lysed by sonication. Cellular debris was removed by centrifugation and filtration of the lysate before applying to the size-exclusion column (SEC-3000; Phenomenex). Green fluorescent proteins were detected with an in-line detector (FP-2020 Plus; Jasco).

## Results and Discussion

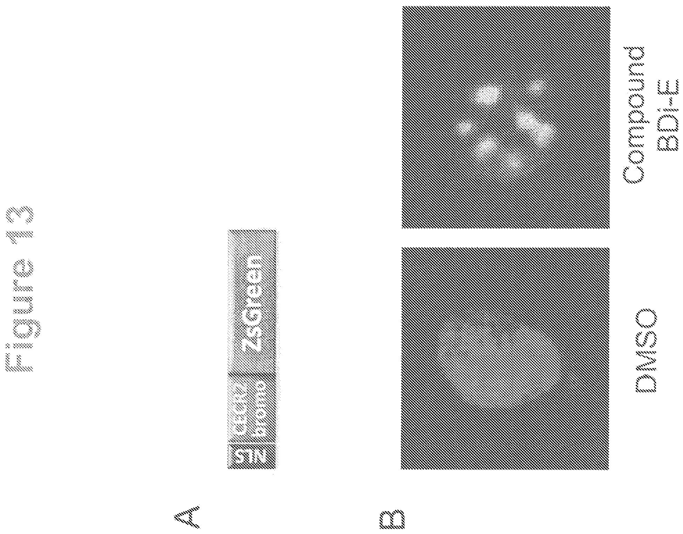

U2OS cell lines stably carrying inducible NLS-CECR2.BD-ZsGreen (Figure 13A) were generated through lentiviral infection and Blasticidin selection. The fusion protein was localized diffusely in the nucleus and showed homogenous distribution in the absence of CECR2 bromodomain inhibitors. Incubation with CECR2 inhibitor (compound BDi-E) resulted in fluorescent dots/foci formation of the ZsGreen-tagged protein (Figure 13B). This indicated the binding of CECR2.BD to chromatin was inhibited by the inhibitors and released to the nucleoplasm. Free NLS-CECR2.BD-ZsGreen fusion protein then aggregated and formed fluorescent dots (foci).

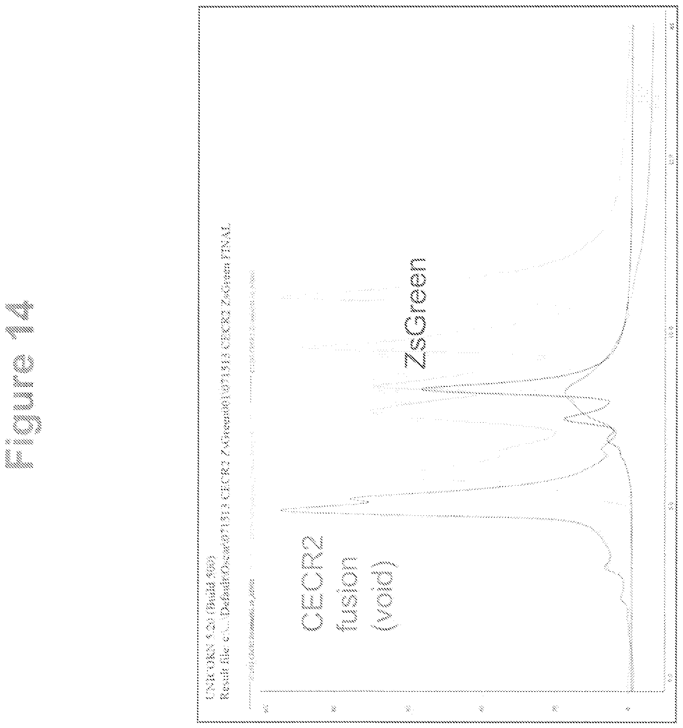

The NLS-CECR2.BD-ZsGreen fusion was expressed in *E. coli* bacteria for comparison to ZsGreen lacking the CECR2 fusion. Cells were lysed and the lysate subjected to gel filtration (sizing column) with fluorescence detection to visualize the ZsGreen proteins. CECR2 fusion protein was eluted in the void volume fraction suggesting a large protein complex (> 700 kD). As expected, ZsGreen protein lacking the CECR2 fusion forms tetramers and dimers but no species eluting in the void volume (Figure 14). These results were consistent with our model (Figure 7) that fluorescent dots contain large protein aggregates.

## Example 6

To explore the concept of using tandem bromodomain modules, but not necessarily other regions of the bromodomain-containing protein, together with tags (e.g., fluorescent protein tags) to establish assay methods to determine inhibitor activity in cells, the following experiment was conducted.

## Methods

Three copies of nuclear localization sequence (DPKKKRKV) (SEQ ID NO:1) were fused to the N-terminal of the coding DNA sequence of two tandem bromodomains of TAF1 (amino acid residues 1406-1640 of NCBI NP_004597.2) followed by the ZsGreen coding sequence. Q5® Site-Directed Mutagenesis Kit (New England BioLabs) was used to generate the bromodomain mutant allele, in which asparagine residues at positions 1481 and 1604 were mutated to tyrosine. Both wild type and mutant fusion expression constructs were cloned into a lentiviral vector, which expresses Tet-On 3G transcription factor under the control of human EF1 promoter. The expression of the NLS-TAF1.BD-ZsGreen, controlled by the inducible TRE3G promoter, was induced by doxycycline. Lentivirus generated with these expression plasmid constructs was used to infect U2OS osteosarcoma cells (ATCC, HTB-96) to establish stable cells under 15 μg/ml Blasticidin selection. Stable cells were plated in 6-well or 96-well plates for microscopic analysis. Cells were treated 2 μg/ml doxycycline for 16 hours before incubation with either DMSO or CECR2 inhibitor for 4 hours prior to fixation with 4% formaldehyde. Zeiss LSM510 inverted confocal microscope or LSM780 confocal microscope with heated stage was used to examine localization of the fluorescent proteins in the nucleus. Acquired images were analyzed with the Zeiss ZEN software.

## Results and Discussion

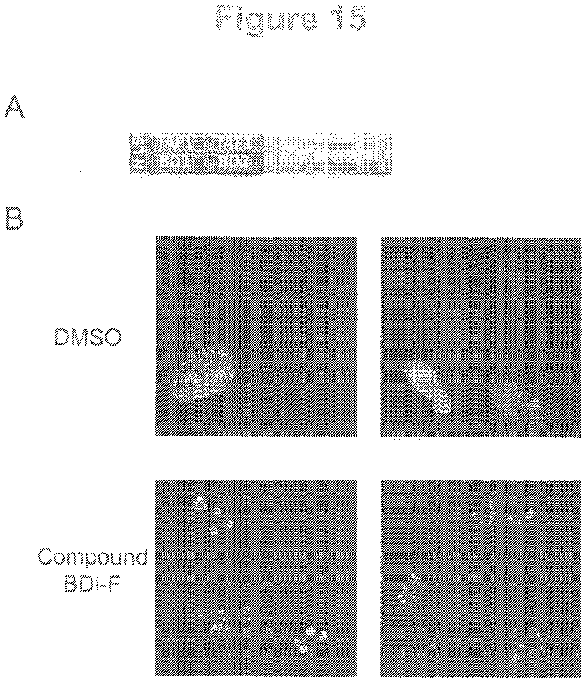

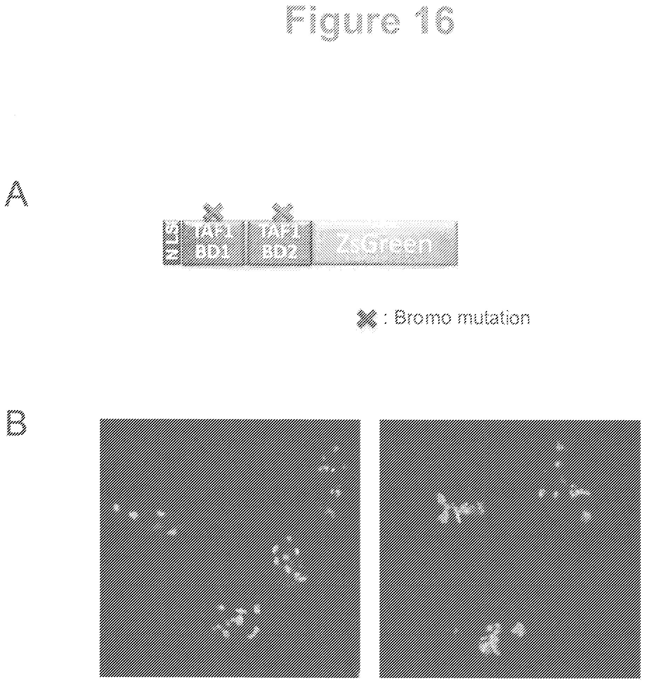

U2OS cells stably carrying inducible NLS-TAF1.BD1.BD2-ZsGreen (Figure 15A) were incubated with doxycycline to induce the expression of the fluorescent protein before treatment with vehicle (DMSO) or TAF1 bromodomain inhibitor for four hours. The ZsGreen-tagged TAF1 bromodomains distributed diffusely in the nucleus in the vehicle control, indicating the fusion protein binds to the chromatin. On the other hand, the tagged protein relocalized and formed fluorescent dots/foci in the presence of TAF1 bromodomain inhibiting compound (BDi-F) (Figure 15B). These results showed that bromodomain inhibitors disrupted the binding of FP-tagged bromodomain proteins to chromatin, and FP-tagged bromodomain proteins formed fluorescent dots/foci after being released from the chromatin. Further, when point mutations that eliminate bromodomain binding ability were introduced to both TAF1 bromodomains of NLS-TAF1.BD1.BD2-ZsGreen (Figure 16A) and the resulting lentiviral construct was used to establish stable U2OS cells, the bromodomain mutant protein formed fluorescent dots/foci even in the absence of bromodomain inhibiting compounds (Figure 16B). Accordingly, FP fusion proteins containing two bromodomain modules formed fluorescent dots/foci in response to bromodomain inhibiting compounds, and this type of engineered cell system could be used to determine the intracellular activity of potential bromodomain inhibiting compounds.

### Example 7

Experiments in this example were designed and conducted to determine the feasibility of using the backbone of full length bromodomain containing protein to develop fluorescent relocalization/dot formation assay for another bromodomain by replacing the bromodomain module of the full-length bromodomain with a chimeric bromodomain module.

### Methods

A fluorescent protein (ZsGreen) was cloned in-frame downstream of the human BRD9 coding DNA sequence (NCBI NP_076413.3), in which the bromodomain coding sequence (amino acid residues 114 - 253) was replaced with the bromodomain sequence of human BAZ2B (NCBI NP_038478.2; amino acid residues 2039 - 2166) or human PCAF/KAT2B (NCBI NP _003875.3; amino acid residues 696 - 820). The chimeric BAZ2B-BRD9-ZsGreen or PCAF-BRD9-ZsGreen fusion sequence was clone into a lentiviral vector, which expresses Tet-On 3G transcription factor under the control of human EF1 promoter. The expression of the ZsGreen-tagged chimeric protein, which were located downstream of the inducible TRE3G promoter, could be induced by doxycycline. Lentivirus generated with these expression plasmid constructs was used to infect U2OS osteosarcoma cells (ATCC, HTB-96) to establish stable cells under 15 μg/ml Blasticidin selection. Stable cells were plated in Nunc™ Lab-Tek™ II chamber slides for confocal microscopy. Cells were treated 2 μg/ml doxycycline for 16 hours to induce expression of the chimeric fluorescent protein. Cells expressing BAZ2B-BRD9-ZsGreen were incubated with either DMSO or BAZ2B inhibitors for 4 hours prior to imaging. Cells expressing PCAF-BRD9-ZsGreen were incubated with either DMSO or PCAF inhibitors for 16 hours prior to imaging. Zeiss LSM510 inverted confocal microscope with heated stage or ImgaeXpress Micro (Molecular Devices) was used to examine localization of the ZsGreen-tagged chimeric proteins. Acquired images were analyzed with the Zeiss ZEN software.

### Results and Discussion

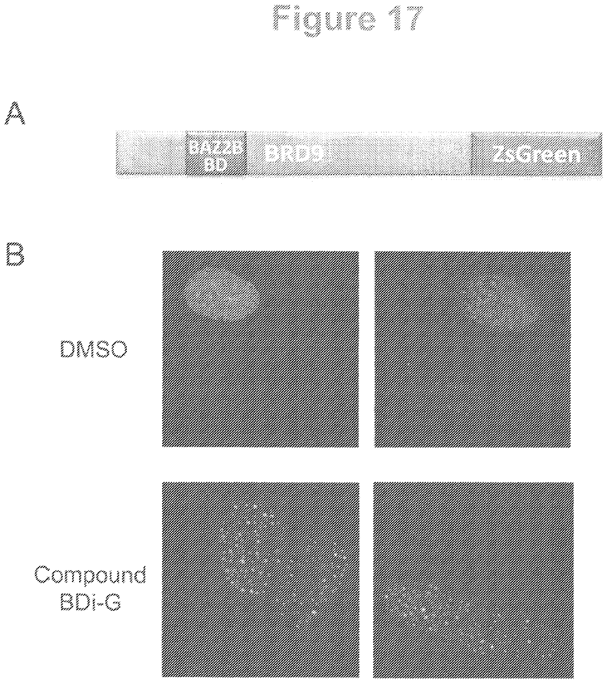

To explore the possibility of using the BRD9-ZsGreen relocalization assay (described in Example 4) to establish an assay system for other bromodomains, a chimeric BRD9-ZsGreen expression construct (called BAZ2B-BRD9-Zs-Green) was generated in which the BRD9 bromodomain was replaced with the bromodomain of BAZ2B. U2OS cells stably carrying inducible BAZ2B-BRD9-ZsGreen (Figure 17A) were incubated with doxycycline to induce the expression of the fluorescent protein followed by treatment with vehicle (DMSO) or bromodomain inhibiting compounds. The nuclear localization sequence of BRD9, which was located upstream of the bromodomain, brought the ZsGreen-tagged chimeric protein to the nucleus. The distribution of this chimeric protein was diffuse in the nucleus in the vehicle control. On the other hand, treatment with BAZ2B bromodomain inhibiting compound (BDi-G) changed the localization of the chimeric protein to form fluorescent dots/foci (Figure 17B). These results showed that BAZ2B inhibitor disrupted the interaction between the BAZ2B-BRD9-ZsGreen protein and the chromatin followed by fluorescent dots/foci formation due to the aggregation property of the ZsGreen tag.

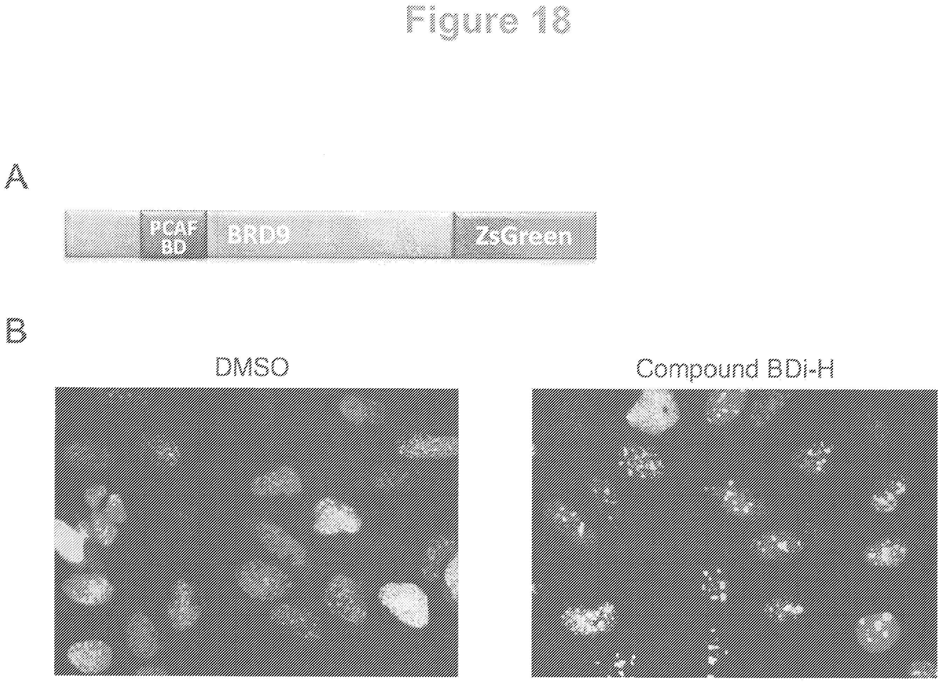

Similarly, the BRD9-ZsGreen backbone was used to generate a PCAF bromodomain chimeric construct (Figure 18A) to determine whether a cellular assay could be established for the PCAF bromodomain to analyze PCAF bromodomain inhibiting compounds. U2OS / PCAF-BRD9-ZsGreen cells were incubation with doxycycline overnight to induce the expression of the ZsGreen tagged chimeric protein follow by vehicle (DMSO) or PCAF inhibiting compound (BDi-H) treatment. The PCAF-BRD9-ZsGreen protein distributed diffusely in the nucleus in the presence of DMSO, and PCAF bromodomain inhibitor resulted in protein relocalization and dots/foci formation (Figure 18B). This result was consistent with ZsGreen-tagged bromodomain proteins relocalizing and forming dots/foci in the presene of bromodomain inhibiting compounds, which inhibit the binding activity of the bromodomain modules. Taken together, experimental data in this example demonstrated that FP-tagged chimeric bromodomain-containing protein containing a replaced bromodomain module from another protein responded to bromodomain inhibiting compounds and were capable of forming fluorescent dots/foci.

## Claims

1. A method for determining whether a test compound is a bromodomain inhibiting compound comprising (a) contacting a cell which contains a fusion protein, wherein a plurality of fusion proteins are capable of forming foci, with the test compound; and (b) determining whether the test compound causes an increase in formation of fusion protein foci, wherein an increase in formation of foci indicates that the test compound is a bromodomain inhibiting compound, wherein each fusion protein comprises at least one bromodomain and a reporter module which is a fluorescent protein that is capable of multimerizing, and
   wherein the reporter module is EGFP, TurboGFP dsRed2, dsRed-Express2 or ZsGreen.

## Patentansprüche

1. Verfahren zum Bestimmen, ob eine Testverbindung eine Bromodomänenhemmende Verbindung ist, das (a) das Kontaktieren einer Zelle, die ein Fusionsprotein enthält, wobei eine Vielzahl an Fusionsproteinen in der Lage ist, Fokusse zu bilden, mit der Testverbindung und (b) das Bestimmen umfasst, ob die Testverbindung eine Erhöhung der Bildung von Fusionsproteinfokussen verursacht, wobei eine Erhöhung der Bildung von Fokussen anzeigt, dass die Testverbindung eine Bromodomänen-hemmende Verbindung ist,
   wobei jedes Fusionsprotein zumindest eine Bromodomäne und ein Reportermodul umfasst, das ein fluoreszierendes Protein ist, das zu Multimerisierung imstande ist, und
   wobei das Reportermodul EGFP, TurboGFP, dsRed2, dsRed-Express2 oder ZsGreen ist.

## Revendications

1. Procédé pour déterminer si un composé de test est un composé inhibiteur de bromodomaine, comprenant les étapes consistant à (a) mettre en contact une cellule qui contient une protéine de fusion, une pluralité de protéines de fusion étant capables de former des foyers, avec le composé de test ; et (b) déterminer si le composé à tester provoque une augmentation de la formation de foyers de protéines de fusion, dans lequel une augmentation de la formation de foyers indique que le composé de test est un composé inhibiteur de bromodomaine,
   dans lequel chaque protéine de fusion comprend au moins un bromodomaine et un module rapporteur qui est une protéine fluorescente capable de multimériser, et
   dans lequel le module rapporteur est EGFP, TurboGFP, dsRed2, dsRed-Express2 ou ZsGreen.

Figure 1

Figure 2

Figure 3

Figure 4

Figure 5

Figure 6

Figure 7

EP 2 970 414 B1

# Figure 8

# Figure 9

Figure 10

A

| BD | BRD9 | ZsGreen |

B

DMSO          Compound BDi-B (10 μ M)          Compound BDi-C (10 μ M)

Figure 11

Figure 12

A

BD  BRD9  mVenus

C

BD  BRD9  mVenus

✖ : Bromo mutation

B

DMSO  Compound BDi-D

D

Mutant BRD9-mVenus

EP 2 970 414 B1

# Figure 13

A

B

DMSO

Compound
BDi-E

Figure 14

# Figure 15

A

B

DMSO

Compound
BDi-F

Figure 16

A

TAF1
BD1 | TAF1
BD2 | Z-Green

✖ : Bromo mutation

B

EP 2 970 414 B1

Figure 17

A

B

DMSO

Compound
BDi-G

EP 2 970 414 B1

Figure 18

A

| PCAF BD | BRD9 | | ZsGreen |

B

DMSO                    Compound BDi-H

EP 2 970 414 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050260186 A
- US 20060104968 A

**Non-patent literature cited in the description**

- **STRUHL K.** *Genes Dev.,* 1989, vol. 12 (5), 599-606
- **KOUZARIDES.** *Cell,* 2007, vol. 128, 693-705
- **MEDZHITOV et al.** *Nat. Rev. Immunol.,* 2009, vol. 9, 692-703
- **PORTELA et al.** *Nat. Biotech.,* 2010, vol. 28, 1057-1068
- **FILLIPAKOPPOULOS et al.** *Cell,* 2012, vol. 149, 214-231
- **JEANMOUGIN F. et al.** *Trends Biochem. Sci.,* 1997, vol. 22 (5), 151-153
- **TAMKUN J.W. et al.** *Cell,* 1992, vol. 7 (3), 561-572
- **PRINJHA et al.** *Trends Pharm. Sci.,* 2012, vol. 33 (3), 146-153
- **MULLER et al.** *Expert Rev.,* September 2011, vol. 13 (29), 1-20
- **SAMBROOK et al.** Molecular Cloning: A Laboratory Manual. 1989
- Oligonucleotide Synthesis. 1984
- Animal Cell Culture. 1987
- Methods in Enzymology. Academic Press, Inc,
- Current Protocols in Molecular Biology. 1987
- PCR: The Polymerase Chain Reaction. 1994
- **PERBAL BERNARD V.** *A Practical Guide to Molecular Cloning,* 1988
- **MIYONG YUN et al.** *Cell Research,* 2011, vol. 21, 564-578
- **FILIPPAKOPOULOS et al.** *Cell,* 2012, vol. 149, 214-231
- **FLATMAN et al.** *J. Chromatogr. B,* 2007, vol. 848, 79-87
- **NEEDLEMAN ; WUNSCH.** *JMB,* 1970, vol. 48, 443
- **OLENYCH et al.** *Cell Biol.,* 2006, 21.5.1-21.5.34
- **NATHAN et al.** *Journal of Cell Science,* 2007, vol. 120, 4247-4260
- **DAY et al.** *Chem. Soc. Rev.,* 2009, vol. 38, 2887-2921
- **SANDBERG et al.** *J. Med. Chem.,* 1998, vol. 41, 2481-91
- **XIE ; SCHULTZ.** *Curr. Opin. Chem. Biol.,* 2005, vol. 9, 548-554
- **HODGSON ; SANDERSON.** *Chem. Soc. Rev,* 2004, vol. 33, 422-430
- **SAMBROOK ; RUSSELL.** Molecular Cloning: A Laboratory Manual. Cold Spring Harbor Laboratory Press, 2001